# EUROPEAN PATENT SPECIFICATION

(11) **EP 4 294 494 B1**
(45) Date of publication and mention of the grant of the patent: **04.11.2026**
(21) Application number: 22708885.3
(22) Date of filing: 22.02.2022
(51) Int. Cl.: A61M 25/10, A61M 25/00, A61M 25/04, A61B 17/12

(54) **LOCO-REGIONAL PERFUSION OF A LIVER**
LOCO-REGIONALE PERFUSION EINER LEBER
PERFUSION LOCORÉGIONALE D'UN FOIE

(30) Priority: 22.02.2021 US 202163151919 P; 20.02.2022 US 202263312029 P
(43) Date of publication of application: 27.12.2023
(73) Proprietor: Dinaqor AG, 8952 Schlieren (CH)
(72) Inventor: HOLZMEISTER, Johannes, London Greater London SW3 5HJ (GB); RICOTTI, Valeria, London Greater London E14 9RU (GB); DEHDASHTIAN, Mark, Irvine, California 92603 (US)
(74) Representative: Maiwald GmbH
(86) International application number: PCT/EP2022/054356
(87) International publication number: WO 2022/175545

(56) References cited:
- EP-A1- 1 098 604
- WO-A1-01/03755
- WO-A1-2007/111495
- CN-U- 203 827 943
- CN-U- 209 392 017
- JP-A- 2017 209 569
- US-A- 4 192 302
- US-A1- 2004 009 151

## Description

### CROSS-REFERENCE TO RELATED APPLICATION(S)

This application claims the benefit of priority of U.S. Provisional Patent Application No. 63/312,029, filed on February 20, 2022, and U.S. Provisional Patent Application Serial No. 63/151,919, filed on February 22, 2021.

### FIELD OF THE INVENTION

The present invention relates to treatment of hepatic diseases, and, in particular, to localized delivery of therapeutic agents to a patient's liver.

### BACKGROUND

Gene therapy and cell therapy techniques in the treatment of various hepatic conditions and diseases, such as hepatitis or hemophilia, have attracted increased attention due to their potential to be uniquely tailored and efficacious in addressing the root cause pathogenesis of various hepatic conditions. Nevertheless, issues related to delivery, including vector efficiency, dose, specificity, and safety remain. As such, there is a need for further research directed to ways of achieving a more targeted, homogenous delivery of drugs suitable for treatment of various hepatic conditions that are also effective, well tolerated, and minimally invasive.

Patent document EP 1 098 604 B1 discloses a device for delivering of a composition to the liver by utilizing the portal vein, whilst patent document WO 01/03755 A1 discloses a perfusion system.

### OBJECTS AND SUMMARY OF THE INVENTION

It is an object of the present invention to provide a system for perfusing a drug in a liver of a patient in a minimally invasive manner.

It is an object of the present invention to provide a system for circulating a perfusate (which may contain one or more of blood or a drug) through a liver of a patient such that the perfusate is isolated from the patient's systemic circulation.

It is an object of the present invention to provide a system for loco-regional delivery of pharmaco-gene therapy.

It is an object of the present invention to provide a system to allow a reduction of the overall dose of a drug delivered to a patient for treating a hepatic condition.

It is an object of the present invention to provide a system to reduce risks and/or adverse immune response to the administration of a drug suitable for treatment of a hepatic condition.

It is an object of the present invention to provide a system to allow for re-dosing and/or dosing a pharmaco-gene therapy drug to patients who possess neutralizing antibodies, e.g., to a gene therapy vector, that would otherwise be unsuitable candidates for receiving such drugs.

It is an object of the present invention to provide a system configured to circulate a perfusate through the liver and isolate the hepatic circulation from the patient's systemic circulation so as to allow a potentially hepatotoxic drug to be introduced into the systemic circulation while preventing or reducing exposure of the drug to the liver.

The above objects and others are met by the present invention by means of a system for performing loco-regional perfusion of a liver of a patient according to claim 1. Preferred embodiments are defined in the dependent claims.

In the following description further reference is made to various methods, none of which fall under the invention as claimed, such methods serving to demonstrate advantages of the inventive system. In some embodiments, the method comprises: positioning a first perfusion catheter in the hepatic artery of the liver; positioning a second perfusion catheter in the portal vein of the liver; positioning one or more recovery catheters in the inferior vena cava of the patient proximal to the liver, such the first perfusion catheter, the second perfusion catheter, and the one or more recovery catheters together with at least one membrane oxygenation device form a closed perfusion circuit through the liver; and causing a perfusate to flow through the closed circuit. In some embodiments, the closed circuit isolates perfusion through the liver from the systemic circulation of the patient.

In some embodiments, positioning the first perfusion catheter in the hepatic artery comprises positioning the first perfusion catheter via the arteria femoralis.

In some embodiments, positioning the second perfusion catheter in the portal vein comprises positioning the second perfusion catheter via the umbilical vein.

In some embodiments, positioning the one or more recovery catheters in the inferior vena cava of the patient comprises positioning a single recovery catheter in each of the left hepatic vein, the middle hepatic vein, and the right hepatic vein. In some embodiments, positioning the one or more recovery catheters comprises positioning a double-balloon catheter with one balloon proximal to the hepatic veins and one balloon distal to the hepatic veins. In some embodiments, a portion of the catheter between the balloons is perforated.

In some embodiments, causing the perfusate to flow through the closed circuit comprises: causing a first portion of the perfusate to pass through a first membrane oxygenation device prior to entering the hepatic artery via the first perfusion catheter; and causing a second portion of the perfusate to pass through a second membrane oxygenation device prior to entering the portal vein via the second perfusion catheter. In some embodiments, the first portion of the perfusate enters the hepatic artery at less than 50% of a total flow rate of the closed circuit, and the second portion of the perfusate enters the portal vein at greater than 50% of the total flow rate of the closed circuit. In some embodiments, the first membrane oxygenation device oxygenates the first portion of the perfusate to full physiological oxygen tension, and the second membrane oxygenation device oxygenates the second portion of the perfusate to less than full physiological oxygen tension. In some embodiments, the second membrane oxygenation device oxygen device oxygenates the second portion of the perfusate to about 50 mmHg to about 80 mmHg of oxygen tension.

In some embodiments, the closed circuit maintains a flow rate of the perfusate at about 1000 mL/min/1.73 m² body surface area to about 1500 mL/min/1.73 m² of body surface area for about 15 min to about 4 hours.

In some embodiments, the method further comprises applying negative pressure at the one or more recovery catheters, such that the negative pressure ranges from about -100 mmHg to 0 mmHg.

In some embodiments, one or more of the first perfusion catheter, the second perfusion catheter, or the one or more recovery catheters are introduced percutaneously.

In some embodiments, the perfusate comprises autologous blood, matched blood from donors, or a combination thereof. In some embodiments, blood components are chosen according to one or more parameters. In some embodiments, the one or more parameters comprise presence or absence of selected antibodies.

In some embodiments, the perfusion is maintained over a duration of about 15 minutes, 30 minutes, 45 minutes, 1 hour, 2 hours, 3 hours, 4 hours, or within any range defined therebetween.

In some embodiments, the drug comprises a therapeutic polynucleotide sequence. In some embodiments, the therapeutic polynucleotide sequence is present in one or more viral vectors. In some embodiments, the one or more viral vectors is selected from the group consisting of an adeno-associated virus, an adenovirus, a retrovirus, a herpes simplex virus, a bovine papilloma virus, a lentiviral vector, a vaccinia virus, a polyoma virus, a sendai virus, orthomyxovirus, paramyxovirus, papovavirus, picornavirus, pox virus, alphavirus, variations thereof, and combinations thereof. In some embodiments, the viral vector is an adeno-associated virus (AAV). In some embodiments, the AAV is one or more of AAV1, AAV2, AAV3, AAV4, AAV5, AAV6, AAV7, AAV8, AAV9, AAV10, AAV11, AAV12, variations thereof, and combinations thereof. In some embodiments, the therapeutic polynucleotide sequence comprises a promoter.

In some embodiments, less than about 30% v/v, less than about 20% v/v, less than about 15% v/v, less than about 10% v/v, less than about 5% v/v, less than about 4% v/v, less than about 3% v/v, less than about 2% v/v, less than about 1% v/v, less than about 0.5% v/v, or substantially no (0% v/v) blood circulated through the closed circuit leaks outside of the closed circuit.

In some embodiments, less than about 30% v/v, less than about 20% v/v, less than about 15%v/v, less than about 10% v/v, less than about 5% v/v, less than about 4% v/v, less than about 3% v/v, less than about 2% v/v, less than about 1% v/v, less than about 0.5% v/v, or substantially no (0% v/v) drug perfused through the closed circuit leaks outside of the closed circuit.

In some embodiments, one or more of the first perfusion catheter, the second perfusion catheter, or the one or more recovery catheters is a balloon catheter.

In another aspect, a method of isolating a liver of a patient from the patient's systemic circulation comprises: positioning a first perfusion catheter in the hepatic artery of the liver; positioning a second perfusion catheter in the portal vein of the liver; positioning one or more recovery catheters in the inferior vena cava of the patient proximal to the liver, such that the first perfusion catheter, the second perfusion catheter, and the one or more recovery catheters together with at least one membrane oxygenation device form a closed perfusion circuit through the liver; and causing a perfusate to flow through the closed circuit, such that the closed circuit isolates the liver from the patient's systemic circulation.

In some embodiments, the method further comprises introducing a drug into the patient's systemic circulation. In some embodiments, the drug is a hepatoxic drug.

In another aspect, a system for performing loco-regional perfusion of a liver of a patient when fluidly coupled thereto comprises: a first perfusion catheter adapted for insertion into the hepatic artery of the liver; a second perfusion catheter adapted for insertion into the portal vein of the liver; one or more recovery catheters adapted for insertion into the inferior vena cava of the patient proximal to the liver; a membrane oxygenation device fluidly coupled to the first perfusion catheter, the second perfusion catheter, the one or more recovery catheters, and an oxygen source, such that the first perfusion catheter, the second perfusion catheter, the one or more recovery catheters, and the membrane oxygenation device together form a closed circuit through the liver that is isolated from the patient's systemic circulation when the first perfusion catheter is inserted into the hepatic artery, the second perfusion catheter is inserted into the portal vein, and the one or more recovery catheters are inserted into the inferior vena cava; and a pump configured to drive fluid flow through the closed circuit.

In some embodiments, the membrane oxygenation device comprises a reservoir configured for injecting a drug into the closed circuit during perfusion.

In some embodiments, the system is adapted to maintain a flow rate of a perfusate through the closed circuit at about 1000 mL/min/1.73 m² body surface area to about 1500 mL/min/1.73 m² of body surface area for about 15 min to about 4 hours.

In another aspect, a system for performing loco-regional perfusion of a liver of a patient comprises: a first perfusion catheter inserted into the hepatic artery of the liver; a second perfusion catheter inserted into the portal vein of the liver; one or more recovery catheters inserted into the inferior vena cava of the patient proximal to the liver; a membrane oxygenation device fluidly coupled to the first perfusion catheter, the second perfusion catheter, the one or more recovery catheters, and an oxygen source, such that the first perfusion catheter, the second perfusion catheter, the one or more recovery catheters, and the membrane oxygenation device together form a closed circuit through the liver that is isolated from the patient's systemic circulation; and a pump configured to drive fluid flow through the closed circuit.

In some embodiments, the membrane oxygenation device comprises a reservoir configured for injecting a drug into the closed circuit during perfusion.

In some embodiments, the system is adapted to maintain a flow rate of a perfusate through the closed circuit at about 1000 mL/min/1.73 m² body surface area to about 1500 mL/min/1.73 m² of body surface area for about 15 min to about 4 hours.

In another aspect, a system of any of the foregoing systems is configured to perform a method of any of the foregoing methods.

The above objects and others are further met by the present invention which in certain embodiments are directed to a loco-regional perfusion system configured to perform any of the aforementioned methods.

### BRIEF DESCRIPTION OF THE DRAWINGS

The above and other features of the present disclosure, their nature, and various advantages will become more apparent upon consideration of the following detailed description, taken in conjunction with the accompanying drawings, in which:
FIG. 1 illustrates a schematic of a first exemplary recovery catheter having a single balloon structure in accordance with at least one embodiment;
FIG. 2 is a photograph of a recovery catheter produced according to an embodiment of the first exemplary recovery catheter;
FIG. 3 illustrates deployment of the first exemplary recovery catheter in accordance with at least one embodiment;
FIG. 4 illustrates deployment of a second exemplary recovery catheter having a single balloon structure in accordance with at least one embodiment;
FIG. 5 illustrates deployment of a third exemplary recovery catheter and a fourth exemplary recovery catheter each having a single balloon structure in accordance with at least one embodiment;
FIG. 6 illustrates deployment of a fifth exemplary recovery catheter having a single balloon structure and a sixth exemplary recovery catheter without a balloon structure in accordance with at least one embodiment;
FIG. 7 illustrates deployment of a seventh exemplary recovery catheter having multiple balloon structures in accordance with at least one embodiment;
FIG. 8 illustrates deployment of an eighth exemplary recovery catheter having a partially covered and recapturable stent structure in accordance with at least one embodiment;
FIG. 9 illustrates deployment of an ninth exemplary recovery catheter having a deployable and retractable stent structure and a balloon structure in accordance with at least one embodiment;
FIG. 10 illustrates deployment of an tenth exemplary recovery catheter having a covered disk-shaped stent structure in accordance with at least one embodiment;
FIG. 11A is a schematic of a first exemplary perfusion catheter having a single balloon structure in accordance with at least one embodiment;
FIG. 11B is a schematic of the balloon structure of the first exemplary perfusion catheter in an expanded state in accordance with at least one embodiment;
FIG. 11C is a schematic of the balloon structure of the first exemplary perfusion catheter in a retracted state in accordance with at least one embodiment;
FIG. 12A is a schematic of a second exemplary perfusion catheter having distal plug in accordance with at least one embodiment;
FIG. 12B is a schematic of the plug of the second exemplary perfusion catheter in accordance with at least one embodiment;
FIG. 12C is a schematic of the plug of the second exemplary perfusion catheter in an extended state in accordance with at least one embodiment;
FIG. 13A is a schematic of a third exemplary perfusion catheter having a distal wedge in accordance with at least one embodiment;
FIG. 13B is a schematic of the wedge of the third exemplary perfusion catheter in accordance with at least one embodiment;
FIG. 13C is a further schematic of the distal end of the third exemplary perfusion catheter in an extended state in accordance with at least one embodiment;
FIG. 14A illustrates deployment of a fourth exemplary perfusion catheter having a partially covered and recapturable stent structure in accordance with at least one embodiment;
FIG. 14B illustrates the stent structure of the fourth exemplary perfusion catheter in a retracted state in accordance with at least one embodiment;
FIG. 14C illustrates the stent structure of the fourth exemplary perfusion catheter in a deployed state in accordance with at least one embodiment;
FIG. 15A illustrates deployment of a fifth exemplary perfusion catheter having a releasable covered braided disk in accordance with at least one embodiment;
FIG. 15B illustrates the braided disk of the fifth exemplary perfusion catheter in a deployed state in accordance with at least one embodiment;
FIG. 16A is a schematic of a sixth exemplary perfusion catheter having a tapered lumen shaft in accordance with at least one embodiment;
FIG. 16B illustrates deployment of the sixth exemplary perfusion catheter in accordance with at least one embodiment;
FIG. 16C illustrates a pre-shaped lumen shaft of the sixth exemplary perfusion catheter in accordance with at least one embodiment;
FIG. 17 illustrates exemplary pre-formed lumen shafts for the exemplary catheters according to the various embodiments;
FIG. 18A depicts an exemplary loco-regional perfusion system in accordance with embodiments of the present disclosure;
FIG. 18B depicts multiple catheters positioned within the hepatic veins in accordance with embodiments of the present disclosure; and
FIG. 19 is a schematic of an exemplary loco-regional perfusion device in accordance with embodiments of the present disclosure.

### DEFINITIONS

As used herein, the singular forms "a," "an," and "the" include plural references unless the context clearly indicates otherwise. Thus, for example, reference to "a drug" includes a single drug as well as a mixture of two or more different drugs; and reference to a "viral vector" includes a single viral vector as well as a mixture of two or more different viral vectors, and the like.

Also as used herein, "about," when used in connection with a measured quantity, refers to the normal variations in that measured quantity, as expected by one of ordinary skill in the art in making the measurement and exercising a level of care commensurate with the objective of measurement and the precision of the measuring equipment. In certain embodiments, the term "about" includes the recited number ±10%, such that "about 10" would include from 9 to 11.

Also as used herein, "polynucleotide" has its ordinary and customary meaning in the art and includes any polymeric nucleic acid such as DNA or RNA molecules, as well as chemical derivatives known to those skilled in the art. Polynucleotides include not only those encoding a therapeutic protein, but also include sequences that can be used to decrease the expression of a targeted nucleic acid sequence using techniques known in the art (e.g., antisense, interfering, or small interfering nucleic acids). Polynucleotides can also be used to initiate or increase the expression of a targeted nucleic acid sequence or the production of a targeted protein within cells of the cardiovascular system. Targeted nucleic acids and proteins include, but are not limited to, nucleic acids and proteins normally found in the targeted tissue, derivatives of such naturally occurring nucleic acids or proteins, naturally occurring nucleic acids or proteins not normally found in the targeted tissue, or synthetic nucleic acids or proteins. One or more polynucleotides can be used in combination, administered simultaneously and/or sequentially, to increase and/or decrease one or more targeted nucleic acid sequences or proteins.

Also as used herein, "perfusion," "perfused," and "perfusing" have their ordinary and customary meaning in the art and refer to administration for a time period (typically a minute or more) that is substantially longer than the art recognized term of "injection" or "bolus injection" (typically less than a minute). The flow rate of the perfusion will depend at least in part on the volume administered.

Also as used herein, "exogenous" nucleic acids or genes are those that do not occur in nature in the vector utilized for nucleic acid transfer; e.g., not naturally found in the viral vector, but the term is not intended to exclude nucleic acids encoding a protein or polypeptide that occurs naturally in the patient or host.

Also as used herein, "hepatic cell" includes any cell of a liver that is involved in maintaining a structure or providing a function of the liver.

Also as used herein, "isolated," "substantially isolated," "largely isolated," and their variants are terms that do not require complete or absolute isolation of the hepatic or systemic circulation; rather, they are intended to mean that a majority, preferably the major part or even substantially all of the specified circulation is isolated. Also as used herein, "partially isolated" refers to any nontrivial portion of the specified circulation being isolated.

Also as used herein, "non-naturally restricted" includes any method of restricting the flow of fluid through a blood vessel, e.g., balloon catheter, sutures, etc., but does not include naturally occurring restriction, e.g., plaque build-up (stenosis). Non-natural restriction includes substantial or total isolation of, for example, the hepatic circulation.

Also as used herein, "minimally invasive" is intended to include any procedure that does not require open surgical access to the liver or vessels closely associated with the liver. Such procedures include the use of endoscopic means to access the liver, and also catheter-based means relying on access via large arteries and veins.

Also as used herein, "adeno-associated virus" or "AAV" encompasses all subtypes, serotypes, and pseudotypes, as well as naturally occurring and recombinant forms. A variety of AAV serotypes and strains are known in the art and are publicly available from sources, such as the ATCC and academic or commercial sources. Alternatively, sequences from AAV serotypes and strains which are published and/or available from a variety of databases may be synthesized using known techniques.

Also as used herein, "serotype" refers to an AAV which is identified by and distinguished from other AAVs based on capsid protein reactivity with defined antisera. There are at least twelve known serotypes of human AAV, including AAV1 through AAV12, however additional serotypes continue to be discovered, and use of newly discovered serotypes are contemplated.

Also as used herein, "pseudotyped" AAV refers to an AAV that contains capsid proteins from one serotype and a viral genome including 5' and 3' inverted terminal repeats (ITRs) of a different or heterologous serotype. A pseudotyped recombinant AAV (rAAV) would be expected to have cell surface binding properties of the capsid serotype and genetic properties consistent with the ITR serotype. A pseudotyped rAAV may comprise AAV capsid proteins, including VP1, VP2, and VP3 capsid proteins, and ITRs from any serotype AAV, including any primate AAV serotype from AAV1 through AAV12, as long as the capsid protein is of a serotype heterologous to the serotype(s) of the ITRs. In a pseudotyped rAAV, the 5' and 3' ITRs may be identical or heterologous. Pseudotyped rAAV are produced using standard techniques described in the art.

Also as used herein, a "chimeric" rAAV vector encompasses an AAV vector comprising heterologous capsid proteins; that is, a rAAV vector may be chimeric with respect to its capsid proteins VP1, VP2, and VP3, such that VP1, VP2, and VP3 are not all of the same serotype AAV. A chimeric AAV as used herein encompasses AAV such that the capsid proteins VP1, VP2, and VP3 differ in serotypes, including for example but not limited to capsid proteins from AAV1 and AAV2; are mixtures of other parvo virus capsid proteins or comprise other virus proteins or other proteins, such as for example, proteins that target delivery of the AAV to desired cells or tissues. A chimeric rAAV as used herein also encompasses an rAAV comprising chimeric 5' and 3' ITRs.

Also as used herein, a "pharmaceutically acceptable excipient or carrier" refers to any inert ingredient in a composition that is combined with an active agent in a formulation. A pharmaceutically acceptable excipient can include, but is not limited to, carbohydrates (such as glucose, sucrose, or dextrans), antioxidants (such as ascorbic acid or glutathione), chelating agents, low-molecular weight proteins, high-molecular weight polymers, gel-forming agents, or other stabilizers and additives. Other examples of a pharmaceutically acceptable carrier include wetting agents, emulsifying agents, dispersing agents, or preservatives, which are particularly useful for preventing the growth or action of microorganisms. Various preservatives are well known and include, for example, phenol and ascorbic acid. Examples of carriers, stabilizers or adjuvants can be found in Remington's Pharmaceutical Sciences, Mack Publishing Company, Philadelphia, Pa., 17th ed. (1985).

Also as used herein, a "patient" refers to a subject, particularly a human (but could also encompass a non-human), who has presented a clinical manifestation of a particular symptom or symptoms suggesting the need for treatment, who is treated prophylactically for a condition, or who has been diagnosed with a condition to be treated.

Also as used herein, a "subject" encompasses the definition of the term "patient" and does not exclude individuals who are otherwise healthy.

Also as used herein, "treatment of" and "treating" include the administration of a drug with the intent to lessen the severity of or prevent a condition, e.g., a hepatic condition or hepatic disease.

Also as used herein, "prevention of" and "preventing" include the avoidance of the onset of a condition, e.g., a hepatic condition or hepatic disease.

Also as used herein, a "condition" or "conditions" refers to those medical conditions, such as a hepatic disease, that can be treated, mitigated, or prevented by administration to a subject of an effective amount of a drug.

Also as used herein, an "effective amount" refers to the amount of a drug that is sufficient to produce a beneficial or desired effect at a level that is readily detectable by a method commonly used for detection of such an effect. In some embodiments, such an effect results in a change of at least 10% from the value of a basal level where the drug is not administered. In other embodiments, the change is at least 20%, 50%, 80%, or an even higher percentage from the basal level. As will be described below, the effective amount of a drug may vary from subject to subject, depending on age, general condition of the subject, the severity of the condition being treated, the particular drug administered, and the like. An appropriate "effective" amount in any individual case may be determined by one of ordinary skill in the art by reference to the pertinent texts and literature and/or by using routine experimentation.

Also as used herein, an "active agent" refers to any material that is intended to produce a therapeutic, prophylactic, or other intended effect, whether or not approved by a government agency for that purpose.

Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to illuminate certain materials and methods and does not pose a limitation on scope. No language in the specification should be construed as indicating any non-claimed element as essential to the practice of the disclosed materials and methods.

### DETAILED DESCRIPTION

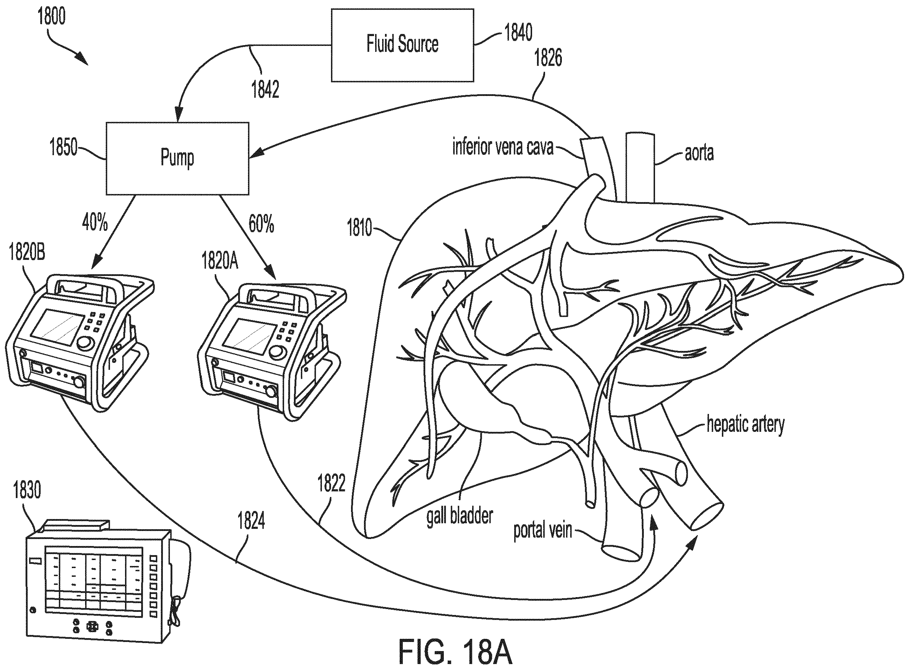

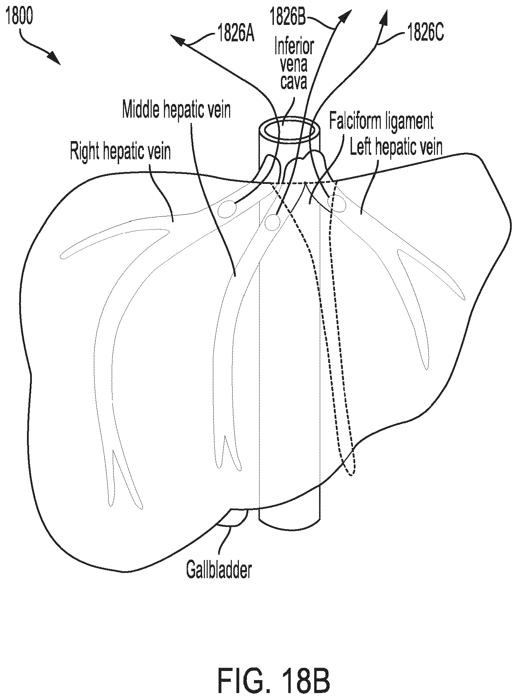

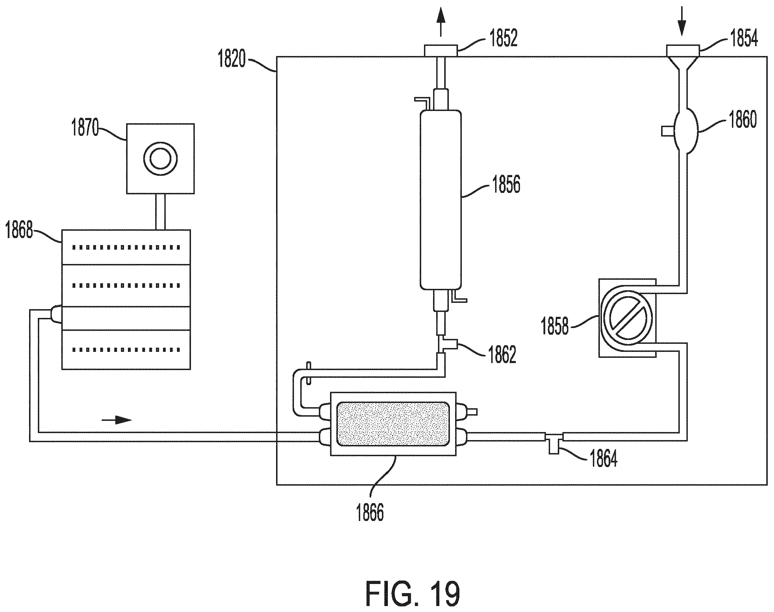

The present invention is directed to systems for treating a hepatic condition in a minimally invasive manner. A method as disclosed, but not falling under the invention as claimed, may comprise isolating a patient's hepatic circulation from the patient's systemic circulation and perfusing a fluid, such as a drug-containing fluid, into the patient's isolated or substantially isolated hepatic circulation. The perfusion may be used to delivery one or more drugs, including, but not limited to, gene therapy vectors, exosomes, nanoparticles, chemotherapy, antibodies, etc., without exposing the systemic circulation and, thus, other organs to the drug(s) chosen. The methods may also be used to isolate the hepatic circulation to allow administration, for example, of a hepatotoxic drug to the patient's systemic circulation in order to protect the liver from adverse effects. Isolation of the patient's hepatic circulation is described in more detail below with reference to FIGS. 18A, 18B, and 19.

Hepatic conditions or diseases that may be treated by the methods disclosed herein may include, without limitations, haemophilia A (factor VIII deficiency) or B (factor IX deficiency), glycogen storage disorder type 1a or type 1b, ornithin transcabamylase deficiency, and phenylketonuria.

In general, total blood flow through the liver in an adult person is about 1000 mL/min. Liver perfusion is different from other organs in that it involves: (1) perfusion with oxygenized blood through the hepatic artery, typically making up about 40% of the total hepatic blood flow; and (2) perfusion through the portal vein, which carries partially oxygenated blood of about 50-80 mmHg oxygen tension and makes up about 60% of total hepatic blood flow. Venous drainage of the liver is effected through the three hepatic veins (the right, middle, and left hepatic veins), which drain into the inferior vena cava. In addition, small hepatic veins drain directly into the inferior vena cava.

To account for the blood flow through the liver described above, in some embodiments, the system includes a first perfusion catheter that may be inserted, for example, via the arteria femoralis and sealed within the hepatic artery with a flow rate appropriate to perfuse and oxygenate the liver for the duration of the procedure. The system may further include a second perfusion catheter that may be inserted, for example, via the umbilical vein and sealed within the portal vein. The system may further include multiple recovery catheters (also referred to as "collection catheters" or "suction catheters") for insertion into the inferior vena cava, which may each be sealed within the left hepatic vein, the middle hepatic vein, and the right hepatic vein. In some embodiments, an additional recovery catheter may be placed into the inferior vena cava if the total hepatic blood flow volume cannot be fully obtained from the three hepatic vein recovery catheters. Alternatively, the multiple recovery catheters may be replaced with a single recovery catheter sealed within the inferior vena cava which may comprise, for example, a first balloon proximal to the hepatic veins and a second balloon distal to the hepatic veins, with the portion of the catheter between the balloons being perforated.

In some embodiments, the system includes one or more extracorporeal membrane oxygenation (ECMO) systems that fluidly connect the venous blood flow from the liver to the arterial and to the portal vein blood flow of the liver, and is capable of oxygenizing the venous blood to various degrees, as required for arterial and portal vein oxygen tension.

In some embodiments, a combined differential double ECMO system may be utilized. Such a system may include, for example, a peristaltic pump coupled to a regulated volume distribution system providing about 40% of the hepatic venous flow to a first ECMO, and about 60% of the hepatic venous flow to a second ECMO. The first ECMO oxygenizes the venous blood to fully physiological oxygen tension and provides the hepatic artery with appropriate flow of physiologically oxygenized blood. The second ECMO provides the portal vein with slightly oxygenated blood (about 50-60 mmHg oxygen tension) at a flow rate sufficient to supply about 60% of the total hepatic flow rate to the portal vein. In some embodiments, one or more access lines may be utilized to allow for drug administration or fluid addition to the first and/or second ECMO.

In some embodiments, the system and method allow for loco-regional perfusion of the liver with a target drug for a duration such as 15 minutes, 30 minutes, 45 minutes, one hour, 2 hours, 3 hours, 4 hours, or for any range defined therebetween. In some embodiments, the system and method allow for selective drug-targeting of the liver with zero or minimal exposure of the systemic circulation and other organs to the drug. In some embodiments, a gene therapy drug may be used to treat a hepatic condition, which may utilize a viral vector (e.g., an adeno-associated virus), naked or encapsulated DNA or RNA molecules, synthetic DNA or RNA analogs (e.g., antisense). In some embodiments, chemotherapy may be used to target a hepatic tumor. In some embodiments, other drugs or biologics/antibodies may be used. In some embodiments, a combination of the aforementioned drugs may be used.

There are a number of advantages to isolating the hepatic circulation of the patient from the systemic circulation of the patient when treating a hepatic condition. These advantages include, but are not limited to: (1) loco-regional delivery of the drug, minimal leakage of the drug to other organs, and reduced overall drug dose; (2) increased targeted drug dose; (3) reduced risks and side-effects; and (4) the possibility to re-dose select patients or to dose patient populations that were not suitable therapy candidates for certain therapies (such as gene therapy with viral vectors to patients who had antibodies to the viral vectors).

### Exemplary Catheter Embodiments

Exemplary recovery catheters and perfusion catheters are now described. The catheters can be configured for the anatomy of any target organ (e.g., a liver), for which LRP is to be performed, as would be appreciated by those of ordinary skill in the art. Moreover, it is to be understood that any of the catheters described as "recovery catheters" could also be used as "perfusion catheters," and vice versa. The embodiments described herein are not limited to LRP of the liver, but may also be used to isolate the circulation of the liver from the systemic circulation, for example, to reduce or prevent exposure of the liver to a drug or other agent introduced into the systemic circulation that may have a deleterious effect on the liver. Those of ordinary skill in the art would appreciate other uses of the catheter embodiments described herein, for example, in applications for which sealing of a blood vessel is desired.

Embodiments of exemplary catheters for use as recovery catheters in an LRP system are now described. In at least one embodiment, the recovery catheters are designed to support a liquid suction flow rate of about 400 mL/min or greater (e.g., about 700 mL/min or greater). For example, in certain embodiments, an exemplary catheter can support an in vitro suction flow rate of about 800 mL/min at about -80 mmHg.

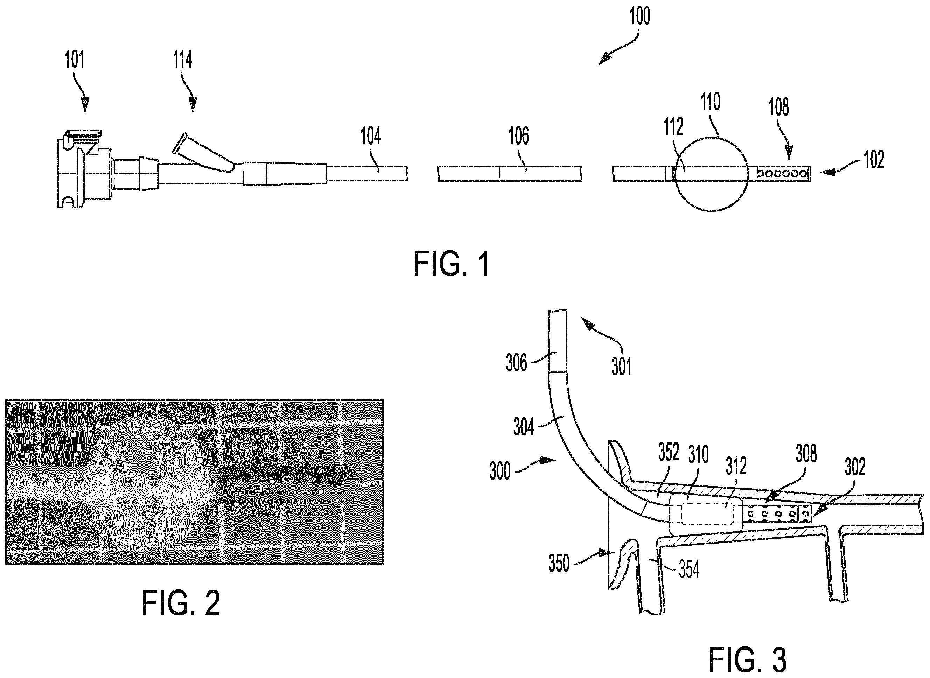

FIGS. 1-10 depict various catheter embodiments suitable for fluid recovery in an LRP system. Any of the catheters depicted in FIGS. 1-10 may be configured to support liquid flow rates (suction or perfusion) of at least about 400 mL/min, at least about 450 mL/min, at least about 500 mL/min, at least about 550 mL/min, at least about 600 mL/min, at least about 650 mL/min, at least about 700 mL/min, at least about 750 mL/min, at least about 800 mL/min, at least about 850 mL/min, at least about 900 mL/min, at least about 950 mL/min, or at least about 1000 mL/min. Each catheter may be compatible with a stearable introducer sheath, which provides stability and directs the distal end of the catheter, and allows for the catheter to create a directed push force. Each catheter may also have a pull wire integrated into its shaft assembly, allowing for sections proximal to the occlusion structure to bend at angles of up to 120° and achieve better tracking and centering of the occlusion structure.

In certain embodiments, one or more of the catheters may be multi-lumen catheters, such as double-lumen catheters. In certain embodiments, the multi-lumen catheters allow for liquid flow (e.g., a perfusate) and enable inflation of one or more balloons. In certain embodiments, one or more of the catheters may be multi-balloon catheters having two or more balloons. In certain embodiments, one or more of the balloons may be deployed or deflated independently.

FIG. 1 illustrates an exemplary catheter 100 having a lumen shaft 104/106 with a proximal end 101 and a distal end 102. The lumen shaft 104/106 can be formed from an outer lumen shaft 104 that at least partially encompasses an inner lumen shaft 106 to expose a distal portion of the inner lumen shaft 106 near the distal end 102. The proximal end 101 includes an outlet structure that can be fluidly coupled to an LRP system. One or more of the outer lumen shaft 104 or the inner lumen shaft 106 may be formed from a durable polymer material such as a polyether block amide (PEBA) material (e.g., commercially available as PEBAX^{®}). In at least one embodiment, an innermost diameter ("inner diameter") of the inner lumen shaft 106 is at least about 4 mm to provide a liquid flow path. In at least one embodiment, the catheter 100 may be designed to include additional lumen shafts.

The catheter 100 includes a tip portion 108 at the distal end 102 and an expandable balloon structure 110 disposed along a portion 112 of the inner lumen shaft 106. In at least one embodiment, the tip portion 108 includes an elongated shaft extending from the balloon structure 110 to the distal end 102. In at least one embodiment, the length of the elongated shaft of the tip portion is from about 2 mm to about 35 mm, about 5 mm to about 30 mm, about 10 mm to about 25 mm, about 15 mm to 25 mm, or within any subrange defined between (e.g., about 2 mm to about 5 mm). In at least one embodiment, the tip portion 108 includes an opening at the distal end 102 and one or more perforations along the elongated shaft. In at least one embodiment, the tip portion is formed from a compliant material that is more flexible than the material of the inner lumen shaft 106.

In at least one embodiment, the inner lumen shaft 106 includes a concentric inner flow path surrounding the liquid flow path. The concentric inner flow path provides a path for gas flow from the balloon structure 110 to a port 114, which can be used to inflate or deflate the balloon depending on the pressure applied at the port 114. In at least one embodiment, an outermost surface of the inner lumen shaft 106 at the portion 112 is removed such that the portion 112 is sealed by the balloon structure 110 to isolate gas flow from the concentric inner flow path to the balloon structure 110. In at least one embodiment, an expanded diameter of the balloon structure is from about 15 mm to about 30 mm, about 15 mm to about 20 mm, about 20 mm to about 25 mm, about 24 mm to about 28 mm, or about 25 mm to about 30 mm.

FIG. 2 is an image of a catheter having a similar structure to the catheter 100 with a balloon in its deployed state. The dimensions of the catheter include: a crossing profile of 19 Fr (6.3 mm); an innermost diameter of 12 Fr (4.0 mm); a usable length of 80 cm; a balloon diameter (when deployed) of 25 mm; and a tip portion length of 20 mm. The lumen shaft can be formed from a polymer material such as PEBAX^{®} 63 that is supported by a strong stainless-steel braid. The balloon can be formed from a compliant thermoplastic/elastomeric material such as ChronoPrene^{™} 25A. The tip portion can be formed from a polymer material such as PEBAX^{®} 35 and can be loaded with a radio marker or a radiopaque filler composition, such as BaSO₄.

FIG. 3 illustrates insertion of an exemplary catheter 300 into a vessel 352 via a larger vessel or chamber 350 (referred to herein as a "vessel") according to at least one embodiment. In the anatomy depicted, blood flow from the vessels 352 and 354 drain into the vessel 350. The catheter 300 may be the same as or similar to the catheter 100, having a proximal end 301, a distal end 302, an inner lumen shaft 304, an outer lumen shaft 306, a tip portion 308, and a balloon structure 310 disposed on a portion 312 of the inner lumen shaft 304. The balloon structure 310 when deployed is compliant enough to adapt to the anatomy of the vessel 352 and occlude the blood flow through the vessel 352 into the vessel 350 without creating excessive force on the tissue. As illustrated in FIG. 3, the catheter 300 is inserted past the vessel 354 so as to avoid occluding the flow from the vessel 354 into the vessel 350.

It is noted that the vessel or chamber 350, the vessel 352, and the vessel 354 are illustrative of the anatomy of, respectively, the right atrium, the coronary sinus, and the middle cardiac vein of a heart to illustrate various types of occlusion techniques for which the exemplary catheters can be utilized. However, they are referred to herein as generic vessels as it is to be understood that the deployment of any of the catheters described herein may be adapted to specific anatomies for target organs (e.g., the liver) in which LRP or occlusion is to be performed. For example, the vessel 350 and the vessel 352 may correspond, respectively, to the inferior vena cava and a hepatic vein of a liver (without the presence of the vessel 354).

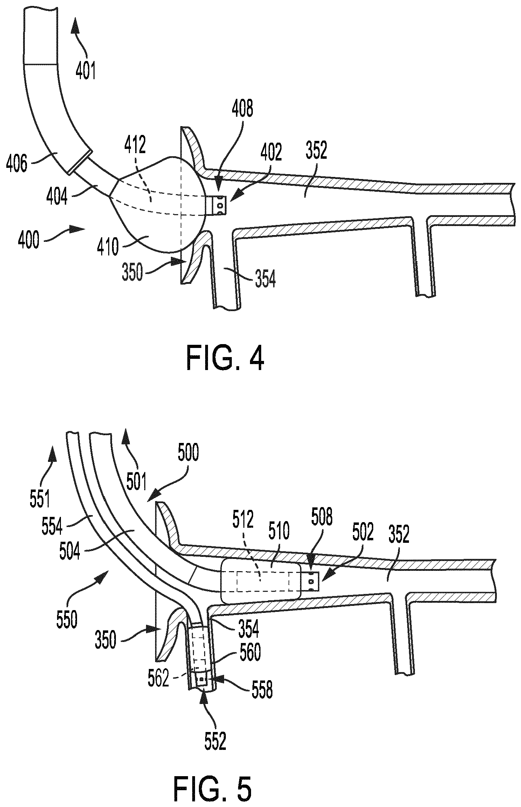

FIGS. 4-10 illustrate other occlusion techniques in accordance with various embodiments of the disclosure. The catheters depicted in FIGS. 4-10 may be similar in certain aspects to the catheters depicted in FIGS. 1-3, for example, in terms of dimensions, materials, or structures.

FIG. 4 illustrates a catheter 400 according to at least one embodiment that is only partially inserted into the vessel 352 such that it abuts the ostium of the vessel 352. The catheter 400 includes a proximal end 401, a distal end 402, an inner lumen shaft 404, an outer lumen shaft 406, a tip portion 408, and a balloon structure 410 disposed on a portion 412 of the inner lumen shaft 404. In at least one embodiment, a diameter of the balloon structure 410 is greater than about 15 mm, greater than about 20 mm, greater than about 25 mm, or greater than about 30 mm when deployed. The tip portion 408 may include, in addition to an opening at the distal end 402, one or more perforations to facilitate flow of blood from the vessel 352 and the vessel 354 into the catheter 400.

In at least one embodiment, during deployment, the outer lumen shaft 406 can be moved distally to abut against the deployed balloon structure 410, resulting in additional pressure by the balloon structure 410 against the ostium of the vessel 352 to further stabilize the position of the catheter 400. In at least another embodiment, a wire structure may be utilized to apply pressure to the balloon structure 410. The wire structure, for example, may have a sinusoidal shape that is deployable to an expanded flower-like structure extending radially from the outer lumen shaft 406 or the inner lumen shaft 404. When brought into contact with the balloon structure 410, the wire structure may produce a more even pressure profile across the surface of the balloon structure 410. Prior to deployment, the wire structure may be covered by the outer lumen shaft 406, or may be covered by an additional lumen outside of the outer lumen shaft 406.

FIG. 5 illustrates the use of a first catheter 500 and a second catheter 550 for separately occluding and draining the vessel 352 and the vessel 354, respectively, according to at least one embodiment. The first catheter 500 includes a proximal end 501, a distal end 502, a lumen shaft 504, a tip portion 508, and a balloon structure 510 disposed on a portion 512 of the lumen shaft 504. Similarly, the second catheter 550 includes a proximal end 551, a distal end 552, a lumen shaft 554, a tip portion 558, and a balloon structure 560 disposed on a portion 562 of the lumen shaft 554. In this configuration, the first catheter 500 is inserted into the vessel 352 such that the balloon structure 510 does not occlude the vessel 354, while the second catheter 550 is inserted directly into the vessel 354. The dimensions of the first catheter 500 and the second catheter 550 may be selected to provide safe and effective occlusion of the vessel 352 and the vessel 354, respectively.

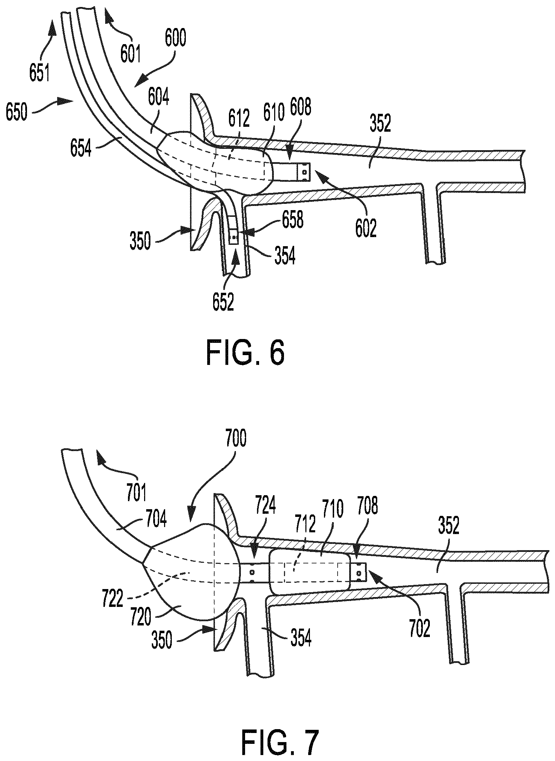

FIG. 6 illustrates a variation of FIG. 5, which uses two catheters with only one having a balloon structure according to at least one embodiment. A first catheter 600 includes a proximal end 601, a distal end 602, a lumen shaft 604, a tip portion 608, and a balloon structure 610 disposed on a portion 612 of the lumen shaft 604. A second catheter 650 includes a proximal end 651, a distal end 652, a lumen shaft 654, and a tip portion 658, and does not include a balloon structure. The first catheter 600 is inserted into the vessel 352 such that a portion of the balloon structure 610 occludes the vessel 354 and is partially within the vessel 350 and the vessel 352. The second catheter 650 is inserted directly into the vessel 354 and is disposed between the vessel wall and the balloon structure 610, which at least partially occludes the vessel 354.

FIG. 7 illustrates the use of a single catheter 700 which includes multiple balloons according to at least one embodiment. The catheter 700 includes a proximal end 701, a distal end 702, a lumen shaft 704, a tip portion 708, a first balloon structure 710 disposed on a first portion 712 of the lumen shaft 704, and a second balloon structure 720 disposed on a second portion 722 of the lumen shaft 704. In at least one embodiment, the catheter 700 is designed for insertion into the vessel 352 such that the first balloon structure 710 occludes the vessel 352, and the second balloon structure 720 abuts the ostium of the vessel 352 to occlude the vessel 354 (and further occlude the vessel 352). An intermediate portion 724 of the lumen shaft 704 between the first balloon structure 710 and the second balloon structure 720 includes one or more perforations to allow drainage of the vessel 354. In at least one embodiment, an expanded diameter of the second balloon structure 720 is greater than an expanded diameter of the first balloon structure 710. In at least one embodiment, the catheter 700 is a multi-lumen catheter designed to allow each balloon to be deployed and deflated independently of each other.

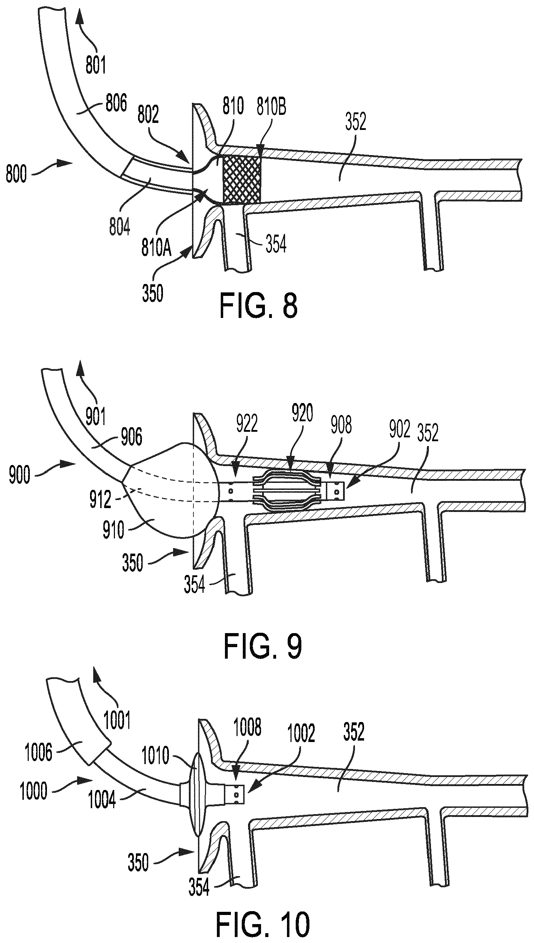

FIG. 8 illustrates a catheter 800 that includes a partially covered and recapturable stent structure 810 according to at least one embodiment. The catheter 800 includes a proximal end 801 and a distal end 802, an inner lumen shaft 804 coupled to the stent structure 810, and an outer lumen shaft 806. Part of the outer lumen shaft 806 is depicted as a cutaway view to illustrate the inner lumen shaft 804 within. The stent structure 810 is depicted in its deployed state, but can be contained within the outer lumen shaft 806 prior to deployment. The stent structure 810 is further depicted as having a proximal covered portion 810A, which may be formed from a flexible and durable polymer material, and a distal uncovered portion 810B. When inserted into the vessel 352, as shown, the covered portion 810A occludes blood flow out of the vessel 352, while the uncovered portion 810B provides structural support within the vessel 352 while allowing blood flow from both the vessel 352 and the vessel 354 directly into the catheter 800. In at least one embodiment, the catheter 800 can be used as a perfusion catheter connected to a supply line.

FIG. 9 illustrates a catheter 900 that includes a deployable and retractable stent structure 920 according to at least one embodiment. The catheter 900 further includes a proximal end 901, a distal end 902, a lumen shaft 906, a tip portion 908, and a balloon structure 910 disposed on a portion 912 of the lumen shaft 906. The catheter 900 can further include an outer lumen shaft (not shown) that substantially encapsulates the stent structure 920 and the balloon structure 910 prior to deployment. Deployment of the stent structure 920 can be performed by moving the outer lumen shaft in a proximal direction, and retraction of the stent structure 920 can be performed by moving the outer lumen shaft in a distal direction. The stent structure 920 may be formed from, for example, stainless-steel, and is disposed between the balloon structure 910 and the tip portion 908. In at least one embodiment, the lumen shaft 906 comprises at least one perforation along a portion 922 between the balloon structure 910 and the stent structure 920 to allow drainage of the vessel 354 into the catheter 900. When inserted into the vessel 352, the balloon structure 910 abuts the ostium of the vessel 352.

FIG. 10 illustrates a catheter 1000 that includes a covered disk-shaped stent structure 1010 according to at least one embodiment. The catheter 1000 further includes a proximal end 1001, a distal end 1002, an outer lumen shaft 1006, an inner lumen shaft 1004, and a tip portion 1008. The stent structure 1010 may be formed from, for example, a stainless-steel stent having a durable polymer covering. The outer lumen shaft 1006 can cover the stent structure 1010 prior to deployment. Once the catheter 1000 is properly positioned, the outer lumen shaft 1006 can be moved in the proximal direction to enable deployment of the stent structure 1010. In at least one embodiment, the stent structure 1010 is coupled to the tip portion 1008, which may be partially contained within the inner lumen shaft 1004 and can be actuatable (using a wire) to deploy the stent structure 1010 when moved in a proximal direction and retract the stent structure 1010 when moved in a distal direction. In at least one embodiment, the stent structure 1010, when deployed, is large enough to occlude the vessel 352 and the vessel 354 when abutted to the ostium of the vessel 352. In at least one embodiment, a diameter of the stent structure 1010 is from about 10 mm to about 30 mm.

Embodiments of exemplary catheters for use as perfusion catheters in an LRP system are now described. In at least one embodiment, the perfusion catheters are designed to support a liquid perfusion flow rate of about 400 mL/min or greater (e.g., about 700 mL/min or greater). In embodiments that utilize multiple perfusion catheters can support a combined flow capacity of 700 mL/min or greater.

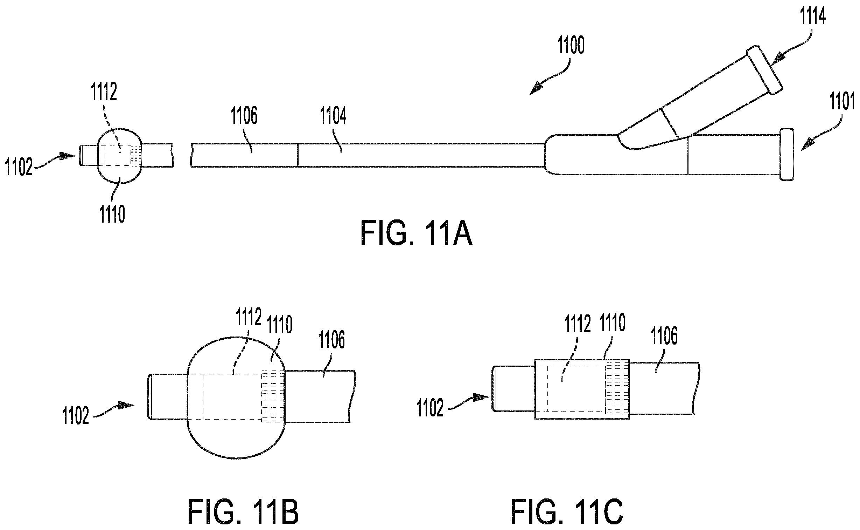

FIGS. 11-16 depict various catheter embodiments suitable for fluid perfusion in an LRP system. Any of the catheters depicted in FIGS. 11-16 may be configured to support liquid flow rates (suction or perfusion) of at least about 400 mL/min, at least about 450 mL/min, at least about 500 mL/min, at least about 550 mL/min, at least about 600 mL/min, at least about 650 mL/min, at least about 700 mL/min, at least about 750 mL/min, at least about 800 mL/min, at least about 850 mL/min, at least about 900 mL/min, at least about 950 mL/min, or at least about 1000 mL/min. Each catheter can be designed to have a smooth profile from a proximal catheter body to a low distal profile, for example, using one or more concentric lumen shafts. In addition, the catheters can be designed to have lumen shafts that are pre-shaped depending on the anatomy in which the LRP procedure is to be performed, which may improve overall stability during use.

In certain embodiments, one or more of the catheters may be multi-lumen catheters, such as double-lumen catheters. In certain embodiments, the multi-lumen catheters allow for liquid flow (e.g., a perfusate) and enable inflation of one or more balloons. In certain embodiments, one or more of the catheters may be multi-balloon catheters having two or more balloons. In certain embodiments, one or more of the balloons may be deployed or deflated independently.

FIGS. 11A-11C illustrate an exemplary catheter 1100 having a lumen shaft 1104/1106 with a proximal end 1101 and a distal end 1102 having an opening from which a perfusate can flow. The lumen shaft 1104/1106 can be formed from an outer lumen shaft 1104 that at least partially encompasses an inner lumen shaft 1106 to expose a distal portion of the inner lumen shaft 1106 near the distal end 1102. The proximal end 1101 includes an outlet structure that can be fluidly coupled to an LRP system. One or more of the outer lumen shaft 1104 or the inner lumen shaft 1106 may be formed from a durable polymer material such as a polyether block amide (PEBA) material (e.g., commercially available as PEBAX^{®}). In at least one embodiment, an innermost diameter of the inner lumen shaft 1106 is at least about 2 mm, at least about 2.5 mm, at least about 3 mm, at least about 3.5 mm, at least about 4 mm, at least about 4.5 mm, or at least about 5 mm to provide a liquid flow path.

The catheter 1100 includes an expandable balloon structure 1110 disposed along a portion 1112 corresponding to the inner lumen shaft 1106 and a tip portion formed by an additional lumen. In at least one embodiment, the inner lumen shaft 1106 includes a concentric inner flow path surrounding the liquid flow path. The concentric inner flow path provides a path for gas flow from the balloon structure 1110 to a port 1114, which can be used to inflate or deflate the balloon structure 1110 depending on the pressure applied at the port 1114. In at least one embodiment, an outermost surface of the inner lumen shaft 1106 at the portion 1112 is removed such that the portion 1112 is sealed by the balloon structure 1110 to isolate gas flow from the concentric inner flow path to the balloon structure 1110. In at least one embodiment, an expanded diameter of the balloon structure 1110 is from about 15 mm to about 30 mm, about 15 mm to about 20 mm, about 20 mm to about 25 mm, about 24 mm to about 28 mm, about 25 mm to about 30 mm, or within any subrange defined therebetween (e.g., about 20 mm to about 28 mm). FIGS. 11B and 11C illustrate the balloon structure 1110 in its deployed and deflated states.

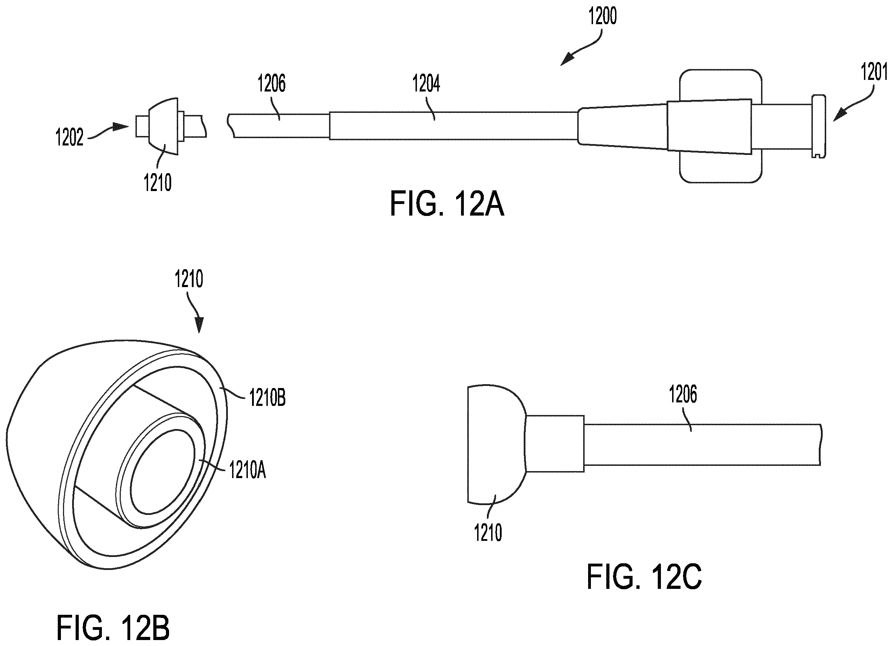

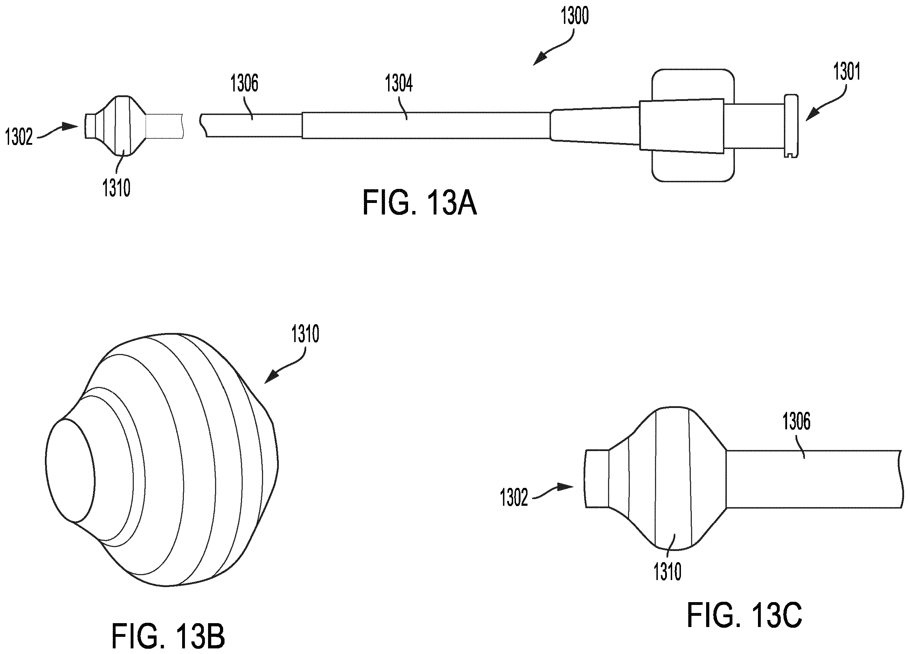

FIGS. 12 and 13 illustrate catheters that include plug and wedge occlusion structures, respectively, that advantageously adapt their shapes to a vessel or ostium, are formed from highly compressible and atraumatic materials for safe introduction and deployment, are shorter in length in comparison to a balloon structure, and do not require an additional lumen for inflation as would a balloon structure.

FIGS. 12A-12C illustrate an exemplary catheter 1200 having a lumen shaft 1204/1206 with a proximal end 1201 and a distal end 1202 having an opening from which a perfusate can flow. The lumen shaft 1204/1206 can be formed from an outer lumen shaft 1204 that at least partially encompasses an inner lumen shaft 1206 to expose a distal portion of the inner lumen shaft 1206 near the distal end 1202. The proximal end 1201 includes an outlet structure that can be fluidly coupled to an LRP system. One or more of the outer lumen shaft 1204 or the inner lumen shaft 1206 may be formed from a durable polymer material such as a polyether block amide (PEBA) material (e.g., commercially available as PEBAX^{®}). In at least one embodiment, an innermost diameter of the inner lumen shaft 1206 is at least about 2 mm, at least about 2.5 mm, at least about 3 mm, at least about 3.5 mm, at least about 4 mm, at least about 4.5 mm, or at least about 5 mm to provide a liquid flow path.

The catheter 1200 further includes a plug 1210 near the distal end 1202. In at least one embodiment, the plug 1210 is formed from a flexible material, such as silicone or a foam material. In at least one embodiment, the plug 1210 includes an inner portion 1210A that fits onto the inner lumen shaft 1206 and a flexible outer portion 1210B shaped to be configurable between a retracted state (FIG. 12A) and an extended state (FIG. 12C) for which the outer portion 1210B extends distally from the distal end 1202. The plug 1210 in FIG. 12A is illustrated as tapering in a distal direction. In at least one embodiment, the plug 1210 may be reversed such that it tapers in a proximal direction. In at least one embodiment, the outer lumen shaft 1204 may be configured to cover the plug 1210 prior to deployment. When utilized as a perfusion catheter, the pressure of arterial blood flow into the hollow space between the inner portion 1210A and the outer portion 1210B of the plug 1210 can help improve the sealing of the catheter 1200 within the vessel in which it is deployed.

FIGS. 13A-13C illustrate an exemplary catheter 1300 having a lumen shaft 1304/1306 with a proximal end 1301 and a distal end 1302 having an opening from which a perfusate can flow. The lumen shaft 1304/1306 can be formed from an outer lumen shaft 1304 that at least partially encompasses an inner lumen shaft 1306 to expose a distal portion of the inner lumen shaft 1306 near the distal end 1302. The proximal end 1301 includes an outlet structure that can be fluidly coupled to an LRP system. One or more of the outer lumen shaft 1304 or the inner lumen shaft 1306 may be formed from a durable polymer material such as a polyether block amide (PEBA) material (e.g., commercially available as PEBAX^{®}). In at least one embodiment, an innermost diameter of the inner lumen shaft 1306 is at least about 2 mm, at least about 2.5 mm, at least about 3 mm, at least about 3.5 mm, at least about 4 mm, at least about 4.5 mm, or at least about 5 mm to provide a liquid flow path.

The catheter 1300 further includes a wedge 1310 near the distal end 1302, which may be shaped to adapt to a vessel or ostium. In at least one embodiment, the wedge 1310 is formed from a flexible material, such as silicone or a foam material. In at least one embodiment, the outer lumen shaft 1304 may be configured to cover the wedge 1310 prior to deployment. When deployed in a vessel, the shape of the wedge can leverage back-up forces from the vessel wall to further enhance stability during occlusion and perfusion of the vessel.

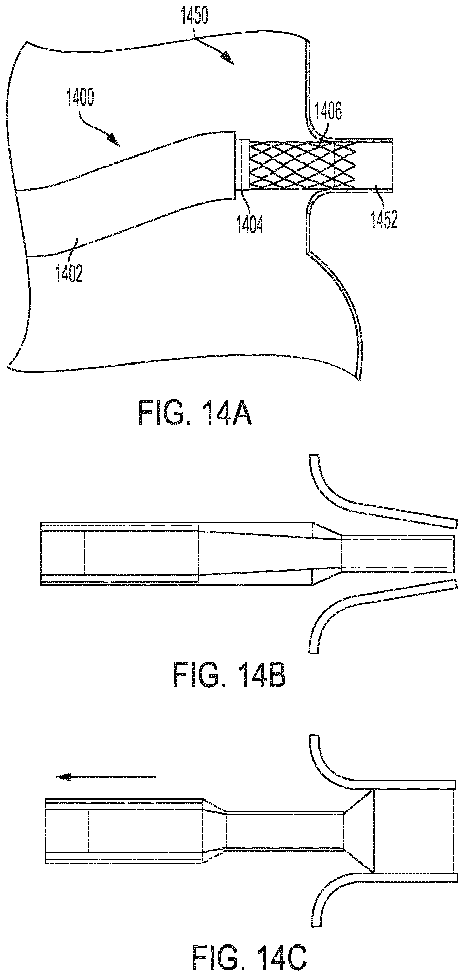

FIGS. 14A-14C illustrate an exemplary catheter 1400 that includes a partially covered and recapturable stent structure 1406 in accordance with at least one embodiment, similar to the catheter 800 described with respect to FIG. 8. The catheter 1400 is illustrated as being inserted into an arterial vessel 1452 via a vessel or chamber 1450. The catheter 1400 includes an outer lumen shaft 1402 and an inner lumen shaft 1404 that is coupled to the stent structure 1406 in certain embodiments. The stent structure 1406 is further depicted as having a proximal covered portion, which may be formed from a flexible and durable polymer material, and a distal uncovered portion. FIGS. 14B and 14C illustrate placement and deployment, respectively, of the stent structure 1406 when inserted into the vessel 1452. Deployment of the stent structure 1406 is performed by moving the outer lumen shaft 1402 in the proximal direction.

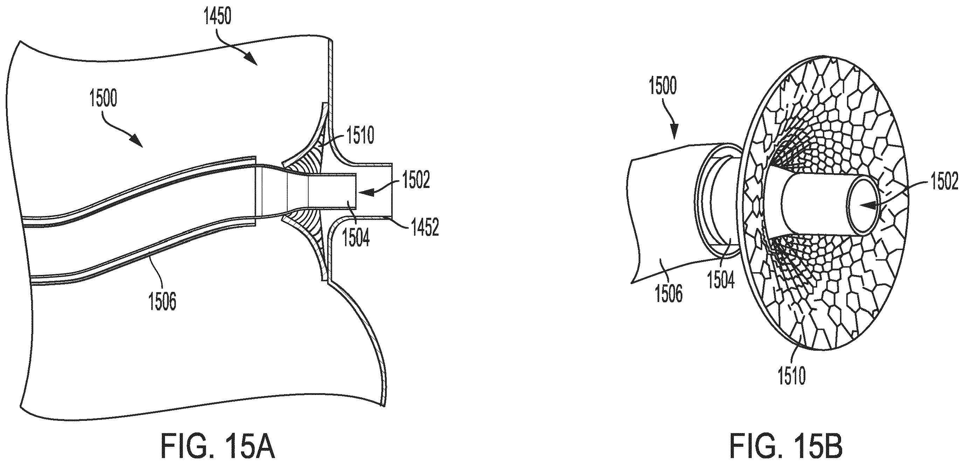

FIGS. 15A and 15B illustrate an exemplary catheter 1500 that includes a releasable covered braided disk 1510, in accordance with at least one embodiment. The catheter 1500 includes an outer lumen shaft 1506 and an inner lumen shaft 1504. The braided disk 1510 is contained within the outer lumen shaft 1506 during placement of the catheter 1500, and can be deployed by moving the outer lumen shaft 1506 in the proximal direction. In certain embodiments, when deployed, the braided disk 1510 does not expand past the distal end 1502, and is used to stabilize the catheter 1500 against the ostium of the vessel 1452 to reduce the risk of stenosis during occlusion of the vessel 1452, while allowing the distal end 1502 to extend into the vessel 1452.

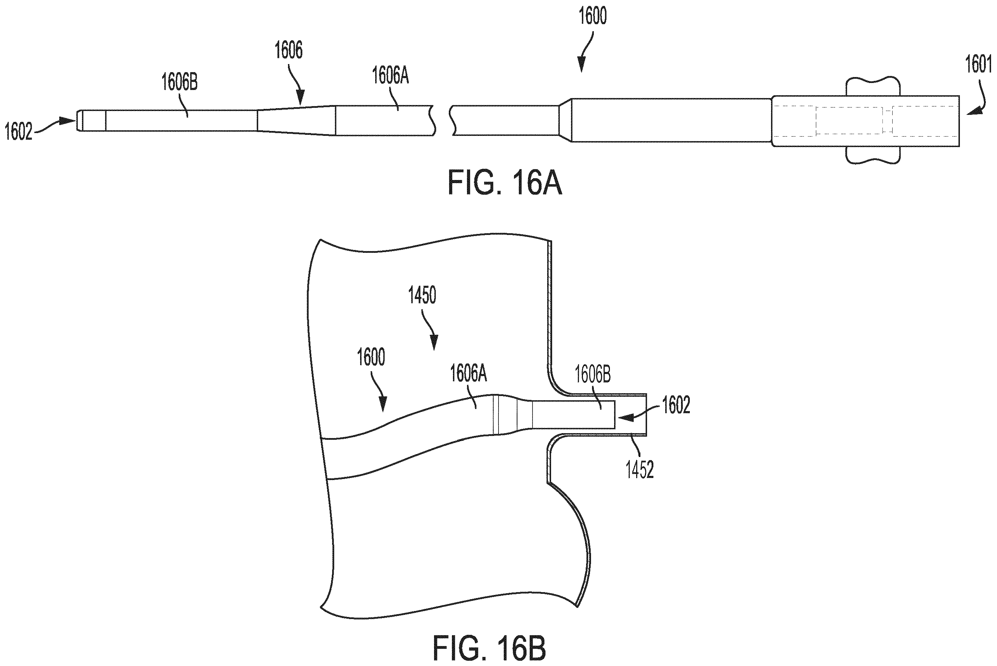

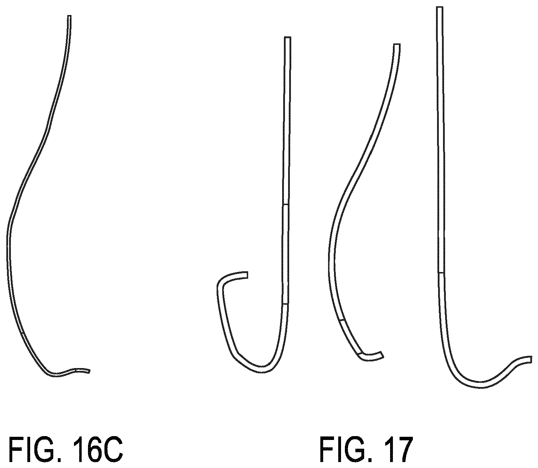

FIGS. 16A-16C illustrate an exemplary catheter 1600 having a lumen shaft 1606 with a proximal end 1601 and a distal end 1602 having an opening from which a perfusate can flow. The proximal end 1601 includes an outlet structure that can be fluidly coupled to an LRP system. The lumen shaft 1604 may be formed from a durable polymer material such as a polyether block amide (PEBA) material (e.g., commercially available as PEBAX^{®}). In at least one embodiment, an innermost diameter of the lumen shaft 1606 is at least about 2 mm, at least about 2.5 mm, at least about 3 mm, at least about 3.5 mm, at least about 4 mm, at least about 4.5 mm, or at least about 5 mm to provide a liquid flow path. In at least one embodiment, a proximal portion 1606A of the lumen shaft 1606 may have a larger diameter than a distal portion 1606B of the lumen shaft 1606, and can taper gradually over a length of the lumen shaft 1606. FIG. 16C illustrates the lumen-shaft in a pre-shaped form to facilitate introduction and placement into a vessel of a target organ.

Examples of pre-shaped catheter lumens are illustrated in FIG. 17. The catheter lumens can be shaped to abut regions of the anatomy when deployed, utilizing back-up forces from the vessel walls to further enhance stability during occlusion and perfusion of the target organ.

### Exemplary LRP System Embodiments

FIG. 18A depicts an exemplary loco-regional perfusion (LRP) system 1800 in accordance with embodiments of the present disclosure. The LRP system 1800 is shown in a closed circuit configuration with a liver 1810. The LRP system 1800 includes membrane oxygenation devices 1820A and 1820B, a blood gas analysis (BGA) monitor 1830 (e.g., fluidly coupled to one or more of the membrane oxygenation devices 1820A or 1820B or various fluid lines), a fluid source 1840, and a pump 1850 (which may be fluidly coupled to the fluid source 1840 via a fluid line 1842). The LRP system 1800 may further include a pressure monitor. The LRP system 1800 may be assembled by positioning a first catheter 1822 ("perfusion catheter") in the portal vein of the liver 1810 via the umbilical vein, a second catheter 1824 ("perfusion catheter") in the hepatic artery of the liver 1810 via the femoral artery, and one or more recovery catheters 1826 ("collection catheters" or "suction catheters") in the hepatic veins or the inferior cava vein via the femoral vein. The first catheter 1822, the second catheter 1824, and the one or more recovery catheters 1826, together with the vasculature of the liver 1810, the membrane oxygenation devices 1820A and 1820B, and one or more optional additional components form a closed circuit. This closed circuit may isolate or substantially isolate the hepatic circulation of the patient from the systemic circulation of the patient. In some embodiments, the membrane oxygenation device 1820A is configured to deliver a perfusate into the portal vein via the first catheter 1822. Similarly, in some embodiments, the membrane oxygenation device 1820B is configured to deliver a perfusate into the portal vein via the second catheter 1824.

The first catheter 1822, the second catheter 1824, and the recovery catheters 1826 may be introduced percutaneously and in a minimally invasive manner. In some embodiments, one or more of the catheters may be introduced via antegrade intubation. In other embodiments, one or more of the catheters may be introduced via retrograde intubation. The first catheter 1822 and the second catheter 1824 may be referred to herein as "drug delivery catheters" and one or more recovery catheters 1826 may be referred to as "drug collection catheters" when the catheters are used for drug delivery to the liver.

The first catheter 1822 and the second catheter 1824 may be infusion catheters with a balloon so as to be wedged, and may optionally include standard guidewires, and are capable of delivering a perfusate to the liver 1810, which may contain, for example, a drug to be delivered to the liver 1810 during loco-regional perfusion.

In some embodiments, the one or more recovery catheters 1826 may be a balloon catheter, such as a Fogarty^{®} catheter, or any other catheter suitable for the purposes discussed herein as will be appreciated by those of ordinary skill in the art. In some embodiments, the first catheter 1822 and the second catheter 1824 may each be a balloon catheter to help reduce leakage. In some embodiments, any of the catheters may be selected from one or more of the catheters described with respect to FIGS. 1-17.

In some embodiments, the recovery catheters 1826 are balloon catheters such that the balloons may be inflated within the hepatic veins to ensure that all the blood circulated through the closed circuit flows through the recovery catheters 1826. As illustrated in FIG. 18B, recovery catheters 1826A, 1826B, and 1826C are each positioned within the left, middle, and right hepatic veins, respectively, and are positioned preferably via the vena femoralis. In some embodiments, the recovery catheters 1826A-1826C are replaced with a single double-balloon catheter inserted into the inferior vena cava with the balloons positioned just below and just above the hepatic veins. In some embodiments, a portion of the catheter between the two balloons may be perforated.

The LRP system 1800 may further comprise one or more additional components, such as, without limitations, one or more pumps (such as the pump 1850), one or more suction mechanisms, one or more perfusates, and combinations thereof. For example, the LRP system 1800 may include a pressure monitor, which in some embodiments is operatively coupled to or part of the membrane oxygenation device 1820. The pressure monitor may be used to control the perfusion rate (i.e., flowrate) and ensure safety by continuously monitoring the arterial pressure. A first pressure sensor and a second pressure sensor, for example, may be co-inserted with the first catheter 1822 and the second catheter 1824, respectively, to measure the pressures within the portal vein and the hepatic artery, respectively. The LRP system 1800 is further depicted as including a BGA monitor 1830 that is operatively coupled to the membrane oxygenation device 1820 to measure, for example, the gas concentrations in the perfusate (e.g., when the perfusate contains blood) prior to perfusion via the first catheter 1822 and the second catheter 1824 and/or after the perfusate is collected by the one or more recovery catheters 1826. The membrane oxygenation devices 1820A and 1820B, as well as additional components, may be placed between the first catheter 1822, the second catheter 1824, and the one or more recovery catheters 1826.

In some embodiments, the pump 1850 (e.g., a peristaltic pump) is used to regulate the fluid volumes delivered to the membrane oxygenation devices 1820A and 1820B, and thus to the portal vein and hepatic artery, respectively. In some embodiments, the pump 1850 causes a portion of the perfusate to enter the portal vein (from the membrane oxygenation device 1820A) at greater than or equal to 50% of a total flow rate of the closed circuit (e.g., about 60%), and causes another portion of the perfusate to enter the hepatic artery (from the membrane oxygenation device 1820B) at less than 50% of the total flow rate of the closed circuit (e.g., about 40%). In some embodiments, the membrane oxygenation device 1820A oxygenates the perfusate entering the portal vein to about 50 mmHg to about 60 mmHg of oxygen tension. In some embodiments, the membrane oxygenation device 1820B oxygenates the perfusate entering the hepatic artery to full physiological oxygen tension.

FIG. 19 is a schematic of the membrane oxygenation device 1820, which may be representative of any of the membrane oxygenation devices 1820A and 1820B. In some embodiments, the membrane oxygenation device 1820 may be used to oxygenate the perfusate, mix the perfusate with other components (e.g., a drug), remove carbon dioxide from the perfusate, and/or push the perfusate into the first catheter 1822. The membrane oxygenation device 1820 may be any commercially available extracorporeal membrane oxygenation (ECMO) device for exchanging oxygen for carbon dioxide contained in the blood.

As illustrated in FIG. 19, the membrane oxygenation device 1820 includes various components including a heat exchanger 1856 (through which the perfusate passes prior to leaving an outlet 1852 and entering the first catheter 1822 or the second catheter 1824), a delivery pump 1858, a reservoir 1860 (for adding a component, such as blood and/or a drug, to the perfusate returning from the one or more recovery catheters 1826 via the pump 1850), sensors 1862 and 1864 at various stages of the closed circuit (e.g., for measuring pressure and/or blood gas content), and a membrane oxygenator 1866. In some embodiments, de-oxygenated blood enters the membrane oxygenator 1866 and is mixed with an oxygen-rich gas. The oxygen-rich gas may be supplied from a gas blender 1868 that may mix oxygen in various ratios with carbon dioxide and nitrogen gas, and is regulated by a gas regulator 1870.

The perfusate may comprise one or more of blood (or its components such as plasma or serum) and/or drug suitable for treatment of the hepatic condition and/or a vehicle such as saline or dextrose solutions. The delivery pump 1858 may deliver the perfusate into the first catheter 1822 or the second catheter 1824, depending on which it is coupled to. In some embodiments, the perfusate may be contained in an IV bag or a syringe and may be administered directly to the first catheter 1822 or the second catheter 1824 with or without the delivery pump 1858.

A suction mechanism may be used to apply negative suction pressure on the one or more recovery catheters 1826 to minimize blood and/or drug leakage outside of the closed circuit. The negative suction pressure may be about -150 mmHg, about -100 mmHg, about -50 mmHg, about -20 mmHg, about -15 mmHg, about -10 mmHg, about -5 mmHg, 0 mmHg, or within a subrange defined by any of these points.

Blood circulated through the closed circuit may be autologous blood, matched blood from donors, or a combination thereof. In some embodiments, blood components, such as serum or plasma, are chosen according to one or more parameters. One of the parameters may be the presence or absence of selected antibodies. For instance, when the drug is one or more viral vectors encompassing a therapeutic nucleic acid sequence, the patient's autologous blood may be screened to determine whether antibodies to the one or more viral vectors are present. Presence of antibodies in the patient's autologous blood may reduce and/or negate altogether the effectiveness of the treatment and/or may result in an undesirable immune response. As such, it may be possible to dilute or replace the patient's autologous blood with a seronegative matched blood from donors in the closed circuit, thereby reducing a patient's immune response to the drug and enhancing the effectiveness of the drug.

While the various components illustrated in FIG. 19 show components that are part of or separate from the membrane oxygenation device 1820, it is to be understood that this schematic is merely illustrative, as one or more of the components may be included in or separate (external) from the membrane oxygenation device 1820.

The LRP system 1800 may be set up and operated as follows: (1) one or more recovery catheters (e.g., the recovery catheters 1826) are carefully placed and tightly sealed in the hepatic veins or just downstream from the hepatic veins in the inferior vena cava to enable the recovery of de-oxygenated venous blood; (2) two perfusion catheters (e.g., the first catheter 1822 and the second catheter 1824) are positioned in each of the portal vein and the hepatic artery in a sealed fashion; (3) each perfusion catheter is connected to a membrane oxygenation device (e.g., the membrane oxygenation devices 1820A and 1820B), and the one or more recovery catheters are then connected to a pump (e.g., the pump 1850) that perfuses the membrane oxygenation devices; (4) operation of the LRP system 1800 is started, and the hepatic artery and portal vein are antegradely perfused with oxygenated blood at their respective physiological levels, while the returning de-oxygenated blood is collected from the hepatic veins via the one or more the recovery catheters using gentle negative pressure; and (5) blood is then directed into the reservoir and is subsequently oxygenated by the membrane oxygenation devices and antegradely re-infused (driven by the delivery pump 1858) into the liver via the perfusion catheters. If a drug (e.g., a vector) is administered, this can be added into the perfusate via the reservoir 1860 after priming with blood or plasma, and blood samples can be taken, or drugs can be applied via the reservoir 1860 during the entire perfusion process.

In some embodiments, diluting or replacing a patient's antibody-containing autologous blood with a seronegative matched blood from donors may result in a reduced adverse immune response and/or improved drug efficacy. For instance, the adversity of a patient's immune response may be reduced by about 10%, by about 20%, by about 30%, by about 40%, by about 50%, by about 60%, by about 70%, by about 80%, by about 90%, or alleviated altogether, upon dilution or replacement of autologous blood with seronegative matched blood from donors as compared to a patient's immune response without autologous blood dilution or replacement. The efficacy of a drug administered may be increased by about 10%, by about 20%, by about 30%, by about 40%, by about 50%, by about 60%, by about 70%, by about 80%, by about 90%, by about 100%, by about 150%, by about 200%, by about 300%, by about 400%, or by about 500%, upon dilution or replacement of autologous blood with seronegative matched blood from donors as compared to the drug's efficacy in a patient without autologous blood dilution or replacement.

In some embodiments, the blood portion of the perfusate may range from about 5 mL to about 5000 mL, from about 50 mL to about 2500 mL, from about 100 mL to about 1000 mL, from about 150 mL to about 500 mL, about 50 mL, about 75 mL, about 100 mL, about 125 mL, about 150 mL, about 175 mL, about 200 mL, about 225 mL, about 250 mL, about 275 mL, about 300 mL, about 325 mL, about 350 mL, about 375 mL, about 400 mL, about 425 mL, about 450 mL, about 475 mL, about 500 mL, about 550 mL, about 600 mL, about 650 mL, about 700 mL, about 750 mL, about 800 mL, about 850 mL, about 900 mL, about 950 mL, or about 1000 mL.

The ratio of autologous blood to blood matched from donors in the blood that is circulated through the closed circuit may be adjusted, as needed, to obtain a blood mixture that would be most receptive to the drug and would generate the least immune response upon introduction of the drug. In some embodiments the ratio may range from about 1:100 to about 100:1, from about 1:80 to about 80:1, from about 1:50 to about 50:1, from about 1:30 to about 30:1, from about 1:20 to about 20:1, from about 1:10 to about 10:1, from about 1:8 to about 8:1, from about 1:5 to about 5:1, from about 1:3 to about 3: 1, or from about 1:2 to about 2:1 of (volume autologous blood) : (volume blood matched from donors).

The flow rate of the perfusate through the closed circuit may be adjusted to match the patient's blood flow rate. As appreciated by one of ordinary skill in the art, the blood flow rate varies from patient to patient, and for any given patient, varies throughout the day. Accordingly, the flow rate of the perfusate circulated through the closed circuit may be adjusted in situ. The flow rate may be measured over the closed circuit. In certain embodiments, the flow rate may be measured with a transonic probe (such as a clamp over tubing). In some embodiments, the flow rate of the perfusate, at any given time during the perfusion, may be within about 20%, within about 15%, within about 10%, within about 8%, within about 5%, within about 3%, within about 2%, within about 1%, or within about 0.5% of the patient's blood flow rate, based on mL/min units. It is important that the flow rate of the perfusate circulated through the closed circuit does not deviate significantly from the patient's own blood flow rate in order to avoid ischemia and/or under perfusion.

Exemplary flow rates for the perfusate circulated through the closed circuit may range, without limitations, from about 75 mL/min to about 750 mL/min, from about 100 mL/min to about 650 mL/min, from about 125 mL/min to about 600 mL/min, from about 150 mL/min to about 500 mL/min, from about 175 mL/min to about 400 mL/min, from about 200 mL/min to about 300 mL/min, about 150 mL/min, about 175 mL/min, about 200 mL/min, about 225 mL/min, about 250 mL/min, about 275 mL/min, about 300 mL/min, about 325 mL/min, or about 350 mL/min. In some embodiments, the system maintains a flow rate of the perfusate in the closed circuit at about 1000 mL/min/1.73 m² body surface area to about 1500 mL/min/1.73 m² of body surface area for about 15 min to about 4 hours.

The perfusate may be circulated through the closed circuit for a duration ranging, without limitations, from about 5 minutes to about 5 hours, from about 15 minutes to about 4 hours, from about 30 minutes to about 3 hours, or from about 1 hour to about 2 hours. In some embodiments, the treatment duration may occur over the span of days, e.g., 1 day, 2 days, 3 days, 4 days, 5 days, 6 days, 7 days, and so on.

With the system disclosed herein, in some embodiments, a higher dose of drug than could otherwise be administered safely through systemic delivery may be administered directly and only to the liver. In some embodiments, a lower overall dose of drug may be required to attain the same therapeutic effect (as was attained with a larger dose that was subjected to systemic circulation or that was subjected to only partial isolation of the hepatic circulation), since there may be substantially no leakage of the perfusate outside of the liver.

In some embodiments, less than about 50% v/v, less than about 40% v/v, less than about 30% v/v, less than about 20% v/v, less than about 15% v/v, less than about 10% v/v, less than about 5% v/v, less than about 4% v/v, less than about 3% v/v, less than about 2% v/v, less than about 1% v/v, less than about 0.5% v/v, or substantially no (0% v/v) perfusate (e.g., blood and/or drug) circulated through the closed circuit leaks outside of the closed circuit during the perfusion process.

The reduced perfusate leakage outside of the closed circuit (as compared to other methods disclosed in the art) may be due to the tight seal formed within the closed circuit and each individual component utilized in the closed circuit.

In certain embodiments, some perfusate leakage from the closed circuit may remain. For instance, up to about 0.5% v/v, about 1% v/v, about 2% v/v, about 3% v/v, about 4% v/v, about 5% v/v, about 10% v/v, about 15% v/v, about 20% v/v, about 30% v/v, about 40% v/v, or about 50% v/v of the perfusate circulated through the closed circuit may leak outside of the closed circuit. Any drug amount lost through leakage of the perfusate may be replaced in the perfusate in order to keep the drug exposure to the liver constant over the calculated exposure time. The calculated exposure time may, in certain embodiments, range from about 5 minutes to about 5 hours, from about 15 minutes to about 4 hours, from about 30 minutes to about 3 hours, from about 1 hour to about 2 hours, or any sub-range in between.

### Therapeutic Compositions

Drugs suitable for treatment of the hepatic condition (i.e., drugs included in the perfusate) may include therapeutic polynucleotide sequences. In some embodiments, the therapeutic polynucleotide sequences may encode to a protein for the treatment of a hepatic condition. The protein for treatment of the hepatic condition may be of human origin or may be derived from different species (e.g., without limitations, mouse, cat, pig or monkey). In some embodiments, the protein encoded by the therapeutic polynucleotide sequence may correspond to a gene expressed in a human liver.

Exemplary proteins may include, without limitations, one or more of Factor VIII, Factor IX, glycogen storage disease (GSD) type 1a (glucose-6-phosphatase), GSD type Ib (glucose-6-phosphate transporter), GSD type III, ornithine transcarbamylase, phenylalanine-4-hydroxylase variants thereof, or combinations thereof. The protein or proteins used may also be functional variants of the proteins mentioned herein and may exhibit a significant amino acid sequence identity compared to the original protein. For instance, the amino acid identity may amount to at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%. In this context, the term "functional variant" means that the variant of the protein is capable of, partially or completely, fulfilling the function of the naturally occurring corresponding protein. Functional variants of a protein may include, for example, proteins that differ from their naturally occurring counterparts by one or more amino acid substitutions, deletions, or additions.

The amino acid substitutions can be conservative or non-conservative. It is preferred that the substitutions are conservative substitutions, i.e., a substitution of an amino acid residue by an amino acid of similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids, which may be used for a conservative substitution, comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine, and lysine. Examples of charged polar (acidic) amino acids comprise aspartic acid and glutamic acid.

Also considered as variants are proteins that differ from their naturally occurring counterparts by one or more (e.g., 2, 3, 4, 5, 10, or 15) additional amino acids. These additional amino acids may be present within the amino acid sequence of the original protein (i.e., as an insertion), or they may be added to one or both termini of the protein. Basically, insertions can take place at any position if the addition of amino acids does not impair the capability of the polypeptide to fulfill the function of the naturally occurring protein in the treated subject. Moreover, variants of proteins also comprise proteins in which, compared to the original polypeptide, one or more amino acids are lacking. Such deletions may affect any amino acid position provided that it does not impair the ability to fulfill the normal function of the protein.

Finally, variants of target proteins also refer to proteins that differ from the naturally occurring protein by structural modifications, such as modified amino acids. Modified amino acids are amino acids which have been modified either by natural processes, such as processing or post-translational modifications, or by chemical modification processes known in the art. Typical amino acid modifications comprise phosphorylation, glycosylation, acetylation, O-linked N-acetylglucosamination, glutathionylation, acylation, branching, ADP ribosylation, crosslinking, disulfide bridge formation, formylation, hydroxylation, carboxylation, methylation, demethylation, amidation, cyclization, and/or covalent or non-covalent bonding to phosphotidylinositol, flavine derivatives, lipoteichonic acids, fatty acids, or lipids.

The therapeutic polynucleotide sequence encoding the target protein may be administered to the subject to be treated in the form of a gene therapy vector, i.e., a nucleic acid construct which comprises the coding sequence, including the translation and termination codons, next to other sequences required for providing expression of the exogenous nucleic acid such as promoters, kozak sequences, polyA signals, and the like.

For example, the gene therapy vector may be part of a mammalian expression system. Useful mammalian expression systems and expression constructs are commercially available. Also, several mammalian expression systems are distributed by different manufacturers and can be employed in the present disclosure, such as plasmid- or viral vector based systems, e.g., LENTI-Smart^{™} (InvivoGen), GenScript^{™} Expression vectors, pAdVAntage^{™} (Promega), ViraPower^{™} Lentiviral, Adenoviral Expression Systems (Invitrogen), and adeno-associated viral expression systems (Cell Biolabs).

Gene therapy vectors for expressing an exogenous therapeutic polynucleotide sequence of the disclosure, can be, for example, a viral or non-viral expression vector, which is suitable for introducing the exogenous therapeutic polynucleotide sequence into a cell for subsequent expression of the protein encoded by said nucleic acid. The expression vector can be an episomal vector, i.e., one that is capable of self-replicating autonomously within the host cell, or an integrating vector, i.e., one which stably incorporates into the genome of the cell. The expression in the host cell can be constitutive or regulated (e.g., inducible).

In a certain embodiment, the gene therapy vector is a viral expression vector. Viral vectors for use in the present disclosure, may comprise a viral genome in which a portion of the native sequence has been deleted in order to introduce a heterogeneous polynucleotide without destroying the infectivity of the virus. Due to the specific interaction between virus components and host cell receptors, viral vectors are highly suitable for efficient transfer of genes into target cells. Suitable viral vectors for facilitating gene transfer into a mammalian cell can be derived from different types of viruses, for example, from an AAV, an adenovirus, a retrovirus, a herpes simplex virus, a bovine papilloma virus, a lentivirus, a vaccinia virus, a polyoma virus, a sendai virus, orthomyxovirus, paramyxovirus, papovavirus, picornavirus, pox virus, alphavirus, or any other viral shuttle suitable for gene therapy, variations thereof, and combinations thereof.

"Adenovirus expression vector" or "adenovirus" is meant to include those constructs containing adenovirus sequences sufficient (a) to support packaging of the therapeutic polynucleotide sequence construct, and/or (b) to ultimately express a tissue and/or cell-specific construct that has been cloned therein. In one embodiment of the disclosure, the expression vector comprises a genetically engineered form of adenovirus. Knowledge of the genetic organization of adenovirus, a 36 kilobase (kb), linear, double-stranded DNA virus, allows substitution of large pieces of adenoviral DNA with foreign sequences up to 7 kb.

Adenovirus growth and manipulation is known to those of skill in the art, and exhibits broad host range in vitro and in vivo. This group of viruses can be obtained in high titers, e.g., 10⁹ to 10¹¹ plaque-forming units per mL, and they are highly infective. The life cycle of adenovirus does not require integration into the host cell genome. The foreign genes delivered by adenovirus vectors are episomal and, therefore, have low genotoxicity to host cells. No side effects have been reported in studies of vaccination with wild-type adenovirus, demonstrating their safety and/or therapeutic potential as in vivo gene transfer vectors.

Retroviruses (also referred to as "retroviral vector") may be chosen as gene delivery vectors due to their ability to integrate their genes into the host genome, transferring a large amount of foreign genetic material, infecting a broad spectrum of species and cell types and for being packaged in special cell-lines.

The retroviral genome contains three genes, gag, pol, and env, that encode for capsid proteins, polymerase enzyme, and envelope components, respectively. A sequence found upstream from the gag gene contains a signal for packaging of the genome into virions. Two long terminal repeat (LTR) sequences are present at the 5' and 3' ends of the viral genome. These contain strong promoter and enhancer sequences and are also required for integration in the host cell genome.

In order to construct a retroviral vector, a nucleic acid encoding a gene of interest is inserted into the viral genome in the place of certain viral sequences to produce a virus that is replication-defective. In order to produce virions, a packaging cell line is constructed containing the gag, pol, and/or env genes but without the LTR and/or packaging components. When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media. The media containing the recombinant retroviruses is then collected, optionally concentrated, and used for gene transfer. Retroviral vectors are able to infect a broad variety of cell types. However, integration and stable expression require the division of host cells.

The retrovirus can be derived from any of the subfamilies. For example, vectors from Murine Sarcoma Virus, Bovine Leukemia, Virus Rous Sarcoma Virus, Murine Leukemia Virus, Mink-Cell Focus-Inducing Virus, Reticuloendotheliosis Virus, or Avian Leukosis Virus can be used. The skilled person will be able to combine portions derived from different retroviruses, such as LTRs, tRNA binding sites, and packaging signals to provide a recombinant retrovirus. These retroviruses are then normally used for producing transduction competent retroviral vector particles. For this purpose, the vectors are introduced into suitable packaging cell lines. Retroviruses can also be constructed for site-specific integration into the DNA of the host cell by incorporating a chimeric integrase enzyme into the retroviral particle.

Because herpes simplex virus (HSV) is neurotropic, it has generated considerable interest in treating nervous system disorders. Moreover, the ability of HSV to establish latent infections in non-dividing neuronal cells without integrating into the host cell chromosome or otherwise altering the host cell's metabolism, along with the existence of a promoter that is active during latency makes HSV an attractive vector. And though much attention has focused on the neurotropic applications of HSV, this vector also can be exploited for other tissues given its wide host range.

Another factor that makes HSV an attractive vector is the size and organization of the genome. Because HSV is large, incorporation of multiple genes or expression cassettes is less problematic than in other smaller viral systems. In addition, the availability of different viral control sequences with varying performance (temporal, strength, etc.) makes it possible to control expression to a greater extent than in other systems. It also is an advantage that the virus has relatively few spliced messages, further easing genetic manipulations.

HSV also is relatively easy to manipulate and can be grown to high titers. Thus, delivery is less of a problem, both in terms of volumes needed to attain sufficient multiplicity of infection (MOI) and in a lessened need for repeat dosing. Avirulent variants of HSV have been developed and are readily available for use in gene therapy contexts.

Lentiviruses are complex retroviruses, which, in addition to the common retroviral genes gag, pol, and env, contain other genes with regulatory or structural function. The higher complexity enables the virus to modulate its life cycle, as in the course of latent infection. Some examples of lentivirus include the Human Immunodeficiency Viruses (HIV-1, HIV-2) and the Simian Immunodeficiency Virus (SIV). Lentiviral vectors have been generated by multiply attenuating the HIV virulence genes, for example, the genes env, vif, vpr, vpu, and nef are deleted making the vector biologically safe.

Lentiviral vectors are plasmid-based or virus-based, and are configured to carry the essential sequences for incorporating foreign nucleic acid, for selection and for transfer of the nucleic acid into a host cell. The gag, pol, and env genes of the vectors of interest also are known in the art. Thus, the relevant genes are cloned into the selected vector and then used to transform the target cell of interest.

Vaccinia virus vectors have been used extensively because of the ease of their construction, relatively high levels of expression obtained, wide host range and large capacity for carrying DNA. Vaccinia contains a linear, double-stranded DNA genome of about 186 kb that exhibits a marked "A-T" preference. Inverted terminal repeats of about 10.5 kb flank the genome. The majority of essential genes appear to map within the central region, which is most highly conserved among poxviruses. Estimated open reading frames in vaccinia virus number from 150 to 200. Although both strands are coding, extensive overlap of reading frames is not common.

At least 25 kb can be inserted into the vaccinia virus genome. Prototypical vaccinia vectors contain transgenes inserted into the viral thymidine kinase gene via homologous recombination. Vectors are selected on the basis of a tk-phenotype. Inclusion of the untranslated leader sequence of encephalomyocarditis virus results in a level of expression that is higher than that of conventional vectors, with the transgenes accumulating at 10% or more of the infected cell's protein in 24 hours.

The empty capsids of papovaviruses, such as the mouse polyoma virus, have received attention as possible vectors for gene transfer. The use of empty polyoma was first described when polyoma DNA and purified empty capsids were incubated in a cell-free system. The DNA of the new particle was protected from the action of pancreatic DNase. The reconstituted particles were used for transferring a transforming polyoma DNA fragment to rat FIII cells. The empty capsids and reconstituted particles consist of all three of the polyoma capsid antigens VP1, VP2, and VP3.

AAVs are parvoviruses belonging to the genus Dependovirus. They are small, nonenveloped, single-stranded DNA viruses which require a helper virus in order to replicate. Co-infection with a helper virus (e.g., adenovirus, herpes virus, or vaccinia virus) is necessary in order to form functionally complete AAV virions. In vitro, in the absence of co-infection with a helper virus, AAV establishes a latent state in which the viral genome exists in an episomal form, but infectious virions are not produced. Subsequent infection by a helper virus "rescues" the genome, allowing it to be replicated and packaged into viral capsids, thereby reconstituting the infectious virion. Recent data indicate that in vivo both wild type AAV and recombinant AAV predominantly exist as large episomal concatemers. In one embodiment, the gene therapy vector used herein is an AAV vector. The AAV vector may be purified, replication incompetent, pseudotyped rAAV particles.

AAV are not associated with any known human diseases, are generally not considered pathogenic, and do not appear to alter the physiological properties of the host cell upon integration. AAV can infect a wide range of host cells, including non-dividing cells, and can infect cells from different species. In contrast to some vectors, which are quickly cleared or inactivated by both cellular and humoral responses, AAV vectors have been shown to induce persistent transgene expression in various tissues in vivo. The persistence of recombinant AAV-mediated transgenes in non-diving cells in vivo may be attributed to the lack of native AAV viral genes and the vector's ITR-linked ability to form episomal concatemers.

AAV is an attractive vector system for use in the cell transduction of the present disclosure, as it has a high frequency of persistence as an episomal concatemer and it can infect non-dividing cells, including cardiomyocytes, thus making it useful for delivery of genes into mammalian cells, for example, in tissue culture and in vivo.

Typically, rAAV is made by cotransfecting a plasmid containing the gene of interest flanked by the two AAV terminal repeats and/or an expression plasmid containing the wild-type AAV coding sequences without the terminal repeats, for example pIM45. The cells are also infected and/or transfected with adenovirus and/or plasmids carrying the adenovirus genes required for AAV helper function. Stocks of rAAV made in such a fashion are contaminated with adenovirus, which must be physically separated from the rAAV particles (for example, by cesium chloride density centrifugation or column chromatography). Alternatively, adenovirus vectors containing the AAV coding regions and/or cell lines containing the AAV coding regions and/or some or all of the adenovirus helper genes could be used. Cell lines carrying the rAAV DNA as an integrated provirus can also be used.

Multiple serotypes of AAV exist in nature, with at least twelve serotypes (AAV1-AAV12). Despite the high degree of homology, the different serotypes have tropisms for different tissues. Upon transfection, AAV elicits only a minor immune reaction (if any) in the host. Therefore, AAV is highly suited for gene therapy approaches.

The present disclosure may be directed in some embodiments to a drug comprising an AAV vector that is one or more of AAV1, AAV2, AAV3, AAV4, AAV5, AAV6, AAV7, AAV8, AAV9, AAV10, AAV11, AAV12, ANC AAV, chimeric AAV derived thereof, variations thereof, and combinations thereof, which will be even better suitable for high efficiency transduction in the tissue of interest. In certain embodiments, the gene therapy vector is an AAV serotype 1 vector. In certain embodiments, the gene therapy vector is an AAV serotype 2 vector. In certain embodiments, the gene therapy vector is an AAV serotype 3 vector. In certain embodiments, the gene therapy vector is an AAV serotype 4 vector. In certain embodiments, the gene therapy vector is an AAV serotype 5 vector. In certain embodiments, the gene therapy vector is an AAV serotype 6 vector. In certain embodiments, the gene therapy vector is an AAV serotype 7 vector. In certain embodiments, the gene therapy vector is an AAV serotype 8 vector. In certain embodiments, the gene therapy vector is an AAV serotype 9 vector. In certain embodiments, the gene therapy vector is an AAV serotype 10 vector. In certain embodiments, the gene therapy vector is an AAV serotype 11 vector. In certain embodiments, the gene therapy vector is an AAV serotype 12 vector.

A suitable dose of AAV for humans may be in the range of about 1x10⁸ vector genomes per kilogram of body weight (vg/kg) to about 3x10¹⁴ vg/kg, about 1x10⁸ vg/kg, about 1x10⁹ vg/kg, about 1x10¹⁰ vg/kg, about 1x10¹¹ vg/kg, about 1x10¹² vg/kg, about 1x10¹³ vg/kg, or about 1x10¹⁴ vg/kg. The total amount of viral particles or DRP is, is about, is at least, is at least about, is not more than, or is not more than about, 5×10¹⁵ vg/kg, 4×10¹⁵ vg/kg, 3×10¹⁵ vg/kg, 2×10¹⁵ vg/kg, 1×10¹⁵ vg/kg, 9×10¹⁴ vg/kg, 8×10¹⁴ vg/kg, 7×10¹⁴ vg/kg, 6×10¹⁴ vg/kg, 5×10¹⁴ vg/kg, 4×10¹⁴ vg/kg, 3×10¹⁴ vg/kg, 2×10¹⁴ vg/kg, 1×10¹⁴ vg/kg, 9×10¹³ vg/kg, 8×10¹³ vg/kg, 7×10¹³ vg/kg, 6×10¹³ vg/kg, 5×10¹³ vg/kg, 4×10¹³ vg/kg, 3×10¹³ vg/kg, 2×10¹³ vg/kg, 1×10¹³ vg/kg, 9×10¹² vg/kg, 8×10¹² vg/kg, 7×10¹² vg/kg, 6×10¹² vg/kg, 5×10¹² vg/kg, 4×10¹² vg/kg, 3×10¹² vg/kg, 2×10¹² vg/kg, 1×10¹² vg/kg, 9×10¹¹ vg/kg, 8×10¹¹ vg/kg, 7×10¹¹ vg/kg, 6×10¹¹ vg/kg, 5×10¹¹ vg/kg, 4×10¹¹ vg/kg, 3×10¹¹ vg/kg, 2×10¹¹ vg/kg, 1×10¹¹ vg/kg, 9×10¹⁰ vg/kg, 8×10¹⁰ vg/kg, 7×10¹⁰ vg/kg, 6×10¹⁰ vg/kg, 5×10¹⁰ vg/kg, 4×10¹⁰ vg/kg, 3×10¹⁰ vg/kg, 2×10¹⁰ vg/kg, 1×10¹⁰ vg/kg, 9×10⁹ vg/kg, 8×10⁹ vg/kg, 7×10⁹ vg/kg, 6×10⁹ vg/kg, 5×10⁹ vg/kg, 4×10⁹ vg/kg, 3×10⁹ vg/kg, 2×10⁹ vg/kg, 1×10⁹ vg/kg, 9×10⁸ vg/kg, 8×10⁸ vg/kg, 7×10⁸ vg/kg, 6×10⁸ vg/kg, 5×10⁸ vg/kg, 4×10⁸ vg/kg, 3×10⁸ vg/kg, 2×10⁸ vg/kg, or 1×10⁸ vg/kg, or falls within a range defined by any two of these values.

With the systems and methods disclosed herein, in some embodiments, a higher dose of drug than could otherwise be administered safely through systemic delivery may be administered directly and only to the liver, since there is substantially no leakage of the perfusate outside of the liver. Alternatively, the loco-regional perfusion of the liver may be used to protect the liver from a systemically circulated hepatotoxic drug, such as a high-dose systemically administered AAV gene therapy, by isolating the liver circulation to prevent the systemically circulating drug from accessing the liver.

Apart from viral vectors, non-viral expression constructs may also be used for introducing a gene encoding a target protein or a functioning variant or fragment thereof into a cell of a patient. Non-viral expression vectors which permit the in vivo expression of protein in the target cell include, for example, a plasmid, a modified RNA, an mRNA, a cDNA, antisense oligomers, DNA-lipid complexes, nanoparticles, exosomes, any other non-viral shuttle suitable for gene therapy, variations thereof, and a combination thereof.

Apart from viral vectors and non-viral expression vectors, nuclease systems may also be used, in conjunction with a vector and/or an electroporation system, to enter into a cell of a patient and introduce therein a gene encoding a target protein or a functioning variant or fragment thereof. Exemplary nuclease systems may include, without limitations, a clustered regularly interspaced short palindromic repeats (CRISPR), a DNA cutting enzyme (e.g., Cas9), meganucleases, TALENs, zinc finger nucleases, any other nuclease system suitable for gene therapy, variations thereof, and a combination thereof. For instance, in one embodiment, one viral vector (e.g., AAV) may be used for a nuclease (e.g., CRISPR) and another viral vector (e.g., AAV) may be used for a DNA cutting enzyme (e.g., Cas9) to introduce both (the nuclease and the DNA cutting enzyme) into a target cell.

Other vector delivery systems which can be employed to deliver a therapeutic polynucleotide sequence encoding a therapeutic gene into cells are receptor-mediated delivery vehicles. These take advantage of the selective uptake of macromolecules by receptor-mediated endocytosis in almost all eukaryotic cells. Because of the cell type-specific distribution of various receptors, the delivery can be highly specific. Receptor-mediated gene targeting vehicles may include two components: a cell receptor-specific ligand and a DNA-binding agent.

Suitable methods for the transfer of non-viral vectors into target cells are, for example, the lipofection method, the calcium-phosphate co-precipitation method, the DEAE-dextran method and direct DNA introduction methods using micro-glass tubes, ultrasound, electroporation, and the like. Prior to the introduction of the vector, the hepatic cells may be treated with a permeabilization agent, such as phosphatidylcholine, streptolysins, sodium caprate, decanoylcarnitine, tartaric acid, lysolecithin, Triton X-100, and the like. Exosomes may also be used to transfer naked DNA or AAV-encapsidated DNA.

A gene therapy vector of the disclosure, may comprise a promoter that is functionally linked to the nucleic acid sequence encoding to the target protein. The promoter sequence should be compact and ensure a strong expression. Preferably, the promoter provides for an expression of the target protein in the liver of the patient that has been treated with the gene therapy vector. In some embodiment, the gene therapy vector comprises a liver-specific promoter which is operably linked to the nucleic acid sequence encoding the target protein. As used herein, a "liver-specific promoter" refers to a promoter whose activity in hepatic cells is at least 2-fold higher than in any other non-hepatic cell type. Preferably, a liver-specific promoter suitable for being used in the vector of the disclosure, has an activity in hepatic cells which is at least 5-fold, at least 10-fold, at least 15-fold, at least 20-fold, at least 25-fold, or at least 50-fold higher compared to its activity in a non-hepatic cell type.

The liver-specific promoter may be a selected human promoter, or a promoter comprising a functionally equivalent sequence having at least about 80%, at least about 90%, at least about 95%, at least about 96%, at least about 97%, at least about 98%, or at least about 99% sequence identity to the selected human promoter. Exemplary non-limiting promoters that may be used are the transthyretin promoter or the thyroxine binding globulin promoter.

The vectors useful in the present disclosure, may have varying transduction efficiencies. As a result, the viral or non-viral vector transduces more than, equal to, or at least about 10%, about 20%, about 30%, about 40%, about 50%, about 55%, about 60%, about 65%, about 70%, about 75%, about 80%, about 85%, about 90%, about 95%, about 99%, or 100% of the cells of the targeted vascular territory. More than one vector (viral or non-viral, or combinations thereof) can be used simultaneously or in sequence. This can be used to transfer more than one polynucleotide, and/or target more than one type of cell. Where multiple vectors or multiple agents are used, more than one transduction/transfection efficiency can result.

Pharmaceutical compositions that contain gene therapy vectors may be prepared either as liquid solutions or suspensions. The pharmaceutical composition of the disclosure, can include commonly used pharmaceutically acceptable excipients, such as diluents and carriers. In particular, the composition comprises a pharmaceutically acceptable carrier, e.g., water, saline, Ringer's solution, or dextrose solution. In addition to the carrier, the pharmaceutical composition may also contain emulsifying agents, pH buffering agents, stabilizers, dyes, and the like.

In certain embodiments, a pharmaceutical composition will comprise a therapeutically effective gene dose, which is a dose that is capable of preventing or treating a hepatic condition in a subject, without being toxic to the subject. Prevention or treatment of the hepatic condition may be assessed as a change in a phenotypic characteristic associated with the hepatic condition with such change being effective to prevent or treat the hepatic condition. Thus, a therapeutically effective gene dose is typically one that, when administered in a physiologically tolerable composition, is sufficient to improve or prevent the pathogenic hepatic phenotype in the treated subject.

### ILLUSTRATIVE PROPHETIC EXAMPLES

The following examples are set forth to assist in understanding the disclosure and should not, of course, be construed as specifically limiting the embodiments described and claimed herein. Such variations of the embodiments, which would be within the purview of those skilled in the art, and changes in formulation or minor changes in experimental design, are to be considered to fall within the scope of the embodiments incorporated herein.

Simple gene replacement by delivering a complete cDNA of a target gene driven by a liver-specific promoter and vectorized in a hepatotropic AAV vector such as AAV5, AAV8, or AAV9, is a suitable treatment approach for diseases where lack of the target gene due to gene mutation is directly causative of the disease phenotype. This treatment approach is particularly feasible for diseases where replacement of less than 100% of the gene product is necessary for a disease rescue. Hepatic genes fulfilling these definitions are, without being limiting, Haemophilia A (factor VIII deficiency), Haemophilia B (factor IX deficiency), GSD type 1a (glucose-6-phosphatase deficiency), GSD type 1b (glucose-6-phosphatase transporter), ornithin-transcarbamylase deficiency, and phenylketonuria (phenylalanine-4-hydroxylase). It is contemplated that all of these diseases can be treated effectively with an AAV-mediated gene replacement approach using the loco-regional liver perfusion method.

In the foregoing description, numerous specific details are set forth, such as specific materials, dimensions, processes parameters, etc., to provide a thorough understanding of the present invention. The particular features, structures, materials, or characteristics may be combined in any suitable manner in one or more embodiments. The words "example" or "exemplary" are used herein to mean serving as an example, instance, or illustration. Any aspect or design described herein as "example" or "exemplary" is not necessarily to be construed as preferred or advantageous over other aspects or designs. Rather, use of the words "example" or "exemplary" is simply intended to present concepts in a concrete fashion. As used in this application, the term "or" is intended to mean an inclusive "or" rather than an exclusive "or". That is, unless specified otherwise, or clear from context, "X includes A or B" is intended to mean any of the natural inclusive permutations. That is, if X includes A; X includes B; or X includes both A and B, then "X includes A or B" is satisfied under any of the foregoing instances. Reference throughout this specification to "an embodiment", "certain embodiments", or "one embodiment" means that a particular feature, structure, or characteristic described in connection with the embodiment is included in at least one embodiment. Thus, the appearances of the phrase "an embodiment", "certain embodiments", or "one embodiment" in various places throughout this specification are not necessarily all referring to the same embodiment.

The present invention has been described with reference to specific exemplary embodiments thereof. The specification and drawings are, accordingly, to be regarded in an illustrative rather than a restrictive sense.

## Claims

1. A system for performing loco-regional perfusion (1800) of a liver (1810) of a patient when fluidly coupled thereto, the system comprising:
a first perfusion catheter adapted for insertion into the hepatic artery (1824) of the liver;
a second perfusion catheter adapted for insertion into the portal vein (1822) of the liver;
one or more recovery catheters (1826) adapted for insertion into the inferior vena cava of the patient proximal to the liver;
a membrane oxygenation device (1820A) fluidly coupled to the first perfusion catheter, the second perfusion catheter, the one or more recovery catheters, and an oxygen source, wherein the membrane oxygenation device comprises a reservoir (1860) containing a drug, the drug comprising a therapeutic polynucleotide sequence present in one or more viral vectors, the reservoir configured for injecting the drug into the closed circuit during perfusion, wherein the first perfusion catheter, the second perfusion catheter, the one or more recovery catheters, and the membrane oxygenation device together form a closed circuit through the liver that is isolated from the patient's systemic circulation when the first perfusion catheter is inserted into the hepatic artery, the second perfusion catheter is inserted into the portal vein, and the one or more recovery catheters are inserted into the inferior vena cava; and
a pump (1850) configured to drive fluid flow of the perfusate through the closed circuit.

2. The system of claim 1, wherein the system is adapted to maintain a flow rate of a perfusate through the closed circuit at about 1000 mL/min/1.73 m² body surface area to about 1500 mL/min/1.73 m² of body surface area for about 15 min to about 4 hours.

3. The system of either claim 1 or claim 2, wherein the one or more viral vectors is selected from the group consisting of an adeno-associated virus, an adenovirus, a retrovirus, a herpes simplex virus, a bovine papilloma virus, a lentiviral vector, a vaccinia virus, a polyoma virus, a sendai virus, orthomyxovirus, paramyxovirus, papovavirus, picornavirus, pox virus, alphavirus, variations thereof, and combinations thereof.

4. The system of claim 3, wherein the viral vector is an adeno-associated virus (AAV).

5. The system of claim 4, wherein the AAV is one or more of AAV1, AAV2, AAV3, AAV4, AAV5, AAV6, AAV7, AAV8, AAV9, AAV10, AAV11, AAV12, variations thereof, and combinations thereof.

6. The system of any one of the preceding claims, wherein the therapeutic polynucleotide sequence comprises a promoter.

7. The system of any one of the preceding claims, wherein the therapeutic polynucleotide sequence is adapted to treat a hepatic condition.

8. The system of any one of the preceding claims, wherein the one or more recovery catheters comprise a double-balloon catheter.

## Patentansprüche

1. System zur Durchführung einer lokoregionalen Perfusion (1800) einer Leber (1810) eines Patienten, wenn es fließend mit dieser gekoppelt ist, wobei das System umfasst:
einen ersten Perfusionskatheter, der zum Einführen in die Leberarterie (1824) der Leber angepasst ist;
einen zweiten Perfusionskatheter, der zum Einführen in die Pfortader (1822) der Leber angepasst ist;
einen oder mehrere Rückgewinnungskatheter (1826), die zum Einführen in die untere Hohlvene des Patienten proximal zur Leber angepasst sind;
eine Membranoxygenierungsvorrichtung (1820A), die fließend mit dem ersten Perfusionskatheter, dem zweiten Perfusionskatheter, dem einen oder den mehreren Rückgewinnungskathetern und einer Sauerstoffquelle verbunden ist, wobei die Membranoxygenierungsvorrichtung ein Reservoir (1860) umfasst, das ein Medikament enthält, wobei das Medikament eine therapeutische Polynukleotidsequenz umfasst, die in einem oder mehreren viralen Vektoren vorliegt, und das Reservoir so konfiguriert ist, dass es das Medikament während der Perfusion in den geschlossenen Kreislauf injiziert, wobei der erste Perfusionskatheter, der zweite Perfusionskatheter, der eine oder die mehreren Rückgewinnungskatheter und die Membranoxygenierungsvorrichtung zusammen einen geschlossenen Kreislauf durch die Leber bilden, der vom systemischen Kreislauf des Patienten isoliert ist, wenn der erste Perfusionskatheter in die Leberarterie, der zweite Perfusionskatheter in die Pfortader und der eine oder die mehreren Rückgewinnungskatheter in die untere Hohlvene eingeführt sind; und
eine Pumpe (1850), die so angepasst ist, dass sie den Flüssigkeitsstrom des Perfusats durch den geschlossenen Kreislauf antreibt.

2. System nach Anspruch 1, wobei das System dazu ausgelegt ist, eine Durchflussrate des Perfusats durch den geschlossenen Kreislauf von etwa 1000 ml/min/1^{,}73^{m2} Körperoberfläche bis etwa 1500 ml/min/1,73^{m2} Körperoberfläche für etwa 15 Minuten bis etwa 4 Stunden aufrechtzuerhalten.

3. System nach Anspruch 1 oder Anspruch 2, wobei der eine oder die mehreren viralen Vektoren aus der Gruppe ausgewählt sind, bestehend aus einem adeno-assoziierten Virus, einem Adenovirus, einem Retrovirus, einem Herpes-simplex-Virus, einem bovinen Papillomavirus, einem lentiviralen Vektor, einem Vacciniavirus, einem Polyomavirus, einem Sendai-Virus, Orthomyxovirus, Paramyxovirus, Papovavirus, Picornavirus, Pockenvirus, Alphavirus, Varianten davon und Kombinationen davon.

4. System nach Anspruch 3, wobei der virale Vektor ein adenoassoziiertes Virus (AAV) ist.

5. System nach Anspruch 4, wobei das AAV eines oder mehrere der folgenden ist: AAV1, AAV2, AAV3, AAV4, AAV5, AAV6, AAV7, AAV8, AAV9, AAV10, AAV11, AAV12, Varianten davon und Kombinationen davon.

6. System nach einem der vorstehenden Ansprüche, wobei die therapeutische Polynukleotidsequenz einen Promotor umfasst.

7. System nach einem der vorstehenden Ansprüche, wobei die therapeutische Polynukleotidsequenz zur Behandlung einer Lebererkrankung hergerichtet ist.

8. System nach einem der vorstehenden Ansprüche, wobei der eine oder die mehreren Rückgewinnungskatheter einen Doppelballonkatheter umfassen.

## Revendications

1. Système destiné à effectuer une perfusion locorégionale (1800) d'un foie (1810) d'un patient lorsqu'il est fluidiquement couplé à celui-ci, le système comprenant :
un premier cathéter de perfusion apte à être inséré dans l'artère hépatique (1824) du foie;
un deuxième cathéter de perfusion apte à être inséré dans la veine porte (1822) du foie ;
un ou plusieurs cathéters de récupération (1826) aptes à être insérés dans la veine cave inférieure du patient, à proximité du foie ;
un dispositif d'oxygénation membranaire (1820A) fluidiquement couplé au premier cathéter de perfusion, au deuxième cathéter de perfusion, à l'un ou aux plusieurs cathéters de récupération, et à une source d'oxygène, sachant que le dispositif d'oxygénation membranaire comprend un réservoir (1860) contenant un médicament, le médicament comprenant une séquence polynucléotidique thérapeutique présente dans un ou plusieurs vecteurs viraux, le réservoir étant configuré pour injecter le médicament dans le circuit fermé pendant la perfusion, sachant que le premier cathéter de perfusion, le deuxième cathéter de perfusion, l'un ou les plusieurs cathéters de récupération, et le dispositif d'oxygénation membranaire forment ensemble un circuit fermé à travers le foie qui est isolé de la circulation systémique du patient lorsque le premier cathéter de perfusion est inséré dans l'artère hépatique, que le deuxième cathéter de perfusion est inséré dans la veine porte, et que l'un ou les plusieurs cathéters de récupération sont insérés dans la veine cave inférieure ; et
une pompe (1850) configurée pour entraîner l'écoulement fluidique du liquide de perfusion à travers le circuit fermé.

2. Le système de la revendication 1, sachant que le système est apte à maintenir un débit d'un liquide de perfusion à travers le circuit fermé d'environ 1 000 mL/min/1,73 m² de surface corporelle à environ 1 500 mL/min/1,73 m² de surface corporelle pendant environ 15 minutes à environ 4 heures.

3. Le système de l'une quelconque des revendications 1 ou 2, sachant que l'un ou les plusieurs vecteurs viraux sont sélectionnés dans le groupe constitué par un virus adéno-associé, un adénovirus, un rétrovirus, un virus de l'herpès simplex, un virus du papillome bovin, un virus lentiviral, un virus de la vaccine, un polyomavirus, un virus Sendai, orthomyxovirus, paramyxovirus, papovavirus, picornavirus, virus de la variole, alphavirus, des variantes de ceux-ci, et des combinaisons de ceux-ci.

4. Le système de la revendication 3, sachant que le vecteur viral est un virus adéno-associé (AAV).

5. Le système de la revendication 4, sachant que l'AAV est un ou plusieurs parmi AAV1, AAV2, AAV3, AAV4, AAV5, AAV6, AAV7, AAV8, AAV9, AAV10, AAV11, AAV12, des variantes de ceux-ci, et des combinaisons de ceux-ci.

6. Le système de l'une quelconque des revendications précédentes, sachant que la séquence polynucléotidique thérapeutique comprend un promoteur.

7. Le système de l'une quelconque des revendications précédentes, sachant que la séquence polynucléotidique thérapeutique est apte à traiter une affection hépatique.

8. Le système de l'une quelconque des revendications précédentes, sachant que l'un ou les plusieurs cathéters de récupération comprennent un cathéter à double ballonnet.
